(19) Europäisches Patentamt
European Patent Office
Office européen des brevets

(11) EP 3 842 800 A1

(12) **EUROPEAN PATENT APPLICATION**
published in accordance with Art. 153(4) EPC

(43) Date of publication:
**30.06.2021 Bulletin 2021/26**

(21) Application number: **19852225.2**

(22) Date of filing: **19.08.2019**

(51) Int Cl.:
***G01N 33/48*** *(2006.01)*
***C07H 5/06*** *(2006.01)*
***G01N 27/62*** *(2021.01)*
***G01N 30/72*** *(2006.01)*
***G01N 33/66*** *(2006.01)*

(86) International application number:
**PCT/JP2019/032218**

(87) International publication number:
**WO 2020/040071 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **21.08.2018 JP 2018155049**

(71) Applicant: **JCR Pharmaceuticals Co., Ltd.**
**Ashiya-shi, Hyogo 659-0021 (JP)**

(72) Inventors:
- **TANAKA, Noboru**
  **Kobe-shi, Hyogo 651-2241 (JP)**
- **KIDA, Sachiho**
  **Kobe-shi, Hyogo 651-2241 (JP)**

(74) Representative: **Balder IP Law, S.L.**
**Paseo de la Castellana 93**
**5a planta**
**28046 Madrid (ES)**

# (54) ANALYTICAL METHOD FOR CHONDROITIN SULFATE

(57) Disclosed is a method for decomposing chondroitin sulfate contained in a sample into disaccharide. In particular disclosed is a method for decomposing chondroitin sulfate contained in a sample into disaccharide by heating the chondroitin sulfate in HCl-methanol containing 2,2-dimethoxypropane at a temperature of 60°C to 90°C for 50 minutes to 180 minutes, optionally in the method, the sample is selected from body fluid, a cell, a tissue, an organ, a cell culture solution, a tissue culture solution, a food, and a feed, or a derived therefrom.

[FIG. 2/7]

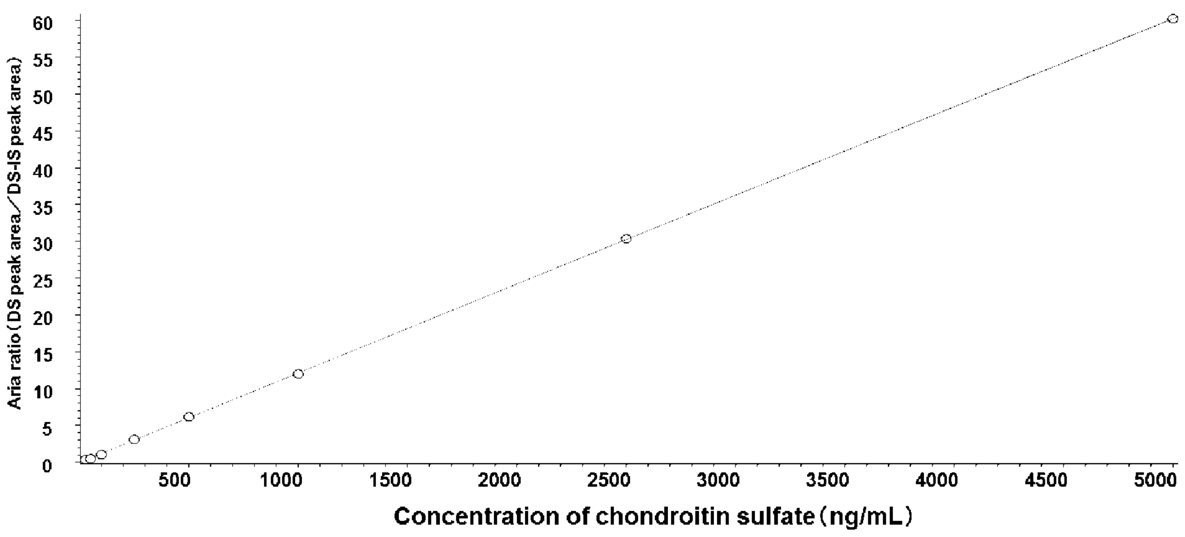


EP 3 842 800 A1

## Description

[TECHNICAL FIELD]

The present invention relates to a method for decomposing chondroitin sulfate into disaccharide, and further relates to a method for measuring the amount of chondroitin sulfate by analyzing the disaccharide with a liquid chromatography-mass spectrometry. In addition, the invention relates to a method for decomposing chondroitin sulfate and heparan sulfate, or dermatan sulfate in addition thereto into disaccharide, and further relates to a method for measuring the amount of chondroitin sulfate and heparan sulfate, or the amount of dermatan sulfate in addition thereto by analyzing the disaccharide with a liquid chromatography-mass spectrometry.

[BACKGROUND ART]

Glycosaminoglycan (GAG) is a group of acidic polysaccharide containing an amino acid. GAG has a long-chain structure in which disaccharides containing amino sugar (glucosamine and galactosamine) and an uronic acid (a glucuronic acid and the like) or galactose are repeatedly bonded. Sugar configuring GAG may be sulfated. Examples of GAG include a hyaluronic acid, chondroitin 4-sulfate, chondroitin 6-sulfate, heparin, heparan sulfate, dermatan sulfate, and keratan sulfate.

An enzyme which specifically degrades a certain type of GAG is present for each type of GAG. For example, β-glucuronidase is an enzyme for hydrolyzing a glycoside bond, and is an enzyme having activity for hydrolyzing a β-glycoside bond by mainly recognizing a -GlcA-GalNAc-structure in chondroitin 4-sulfate. In a case where a part or all of the activity of the enzyme is genetically lost, a Sly syndrome (mucopolysaccharidosis type VII) is developed. Chondroitin 4-sulfate and the like are accumulated in the body of a patient of the Sly syndrome, and thus, various symptoms such as body deformation and hypophrenia occur.

In addition, a Morquio syndrome and the like are known as a disorder due to a genetic defect of the enzyme for decomposing GAG, in which chondroitin 4-sulfate and the like are accumulated in the body.

The disorder caused by the defect of the enzyme for decomposing GAG is collectively referred to as mucopolysaccharidosis. GAG is abnormally accumulated in the tissue of a patient of the mucopolysaccharidosis. For example, GAG containing chondroitin 4-sulfate is accumulated in the body of the patient of the Sly syndrome. Accordingly, the effect of a medical agent for the mucopolysaccharidosis, for example, can be quantified by measuring a change in the amount of GAG present in the tissue. In addition, the patient of the mucopolysaccharidosis can be identified by measuring the amount of GAG present in the tissue.

A method for obtaining fluorescence labeling disaccharides by decomposing GAG contained in a sample with a reductive amination reaction and for analyzing the fluorescence labeling disaccharides with liquid chromatography has been known as an analytical method for GAG (Patent Document 1). In addition, a method for decomposing GAG contained in the sample into disaccharides with methanolysis and analyzing the disaccharides by using a mass spectrometer directly connected to liquid chromatography has been known (Non-Patent Documents 1 to 3).

[PRIOR ART DOCUMENTS]

[Patent Document]

[Patent Document 1] JP 2012-108056 A

[Non-patent Documents]

[Non-Patent Document 1]: Auray-Blais C. et al., Mol Genet Metab. 102. 49-56 (2011)
[Non-Patent Document 2]: Auray-Blais C. et al., Clin Chim Acta. 413. 771-8 (2012)
[Non-Patent Document 3]: Zang H. et al., Clin Chem. 57. 1005-12 (2011)


[SUMMARY OF INVENTION]

[TECHNICAL PROBLEM]

An objective of the invention is to provide a method for decomposing chondroitin sulfate into disaccharides configuring the chondroitin sulfate, and a method for analyzing and measuring disaccharides obtained by decomposing

chondroitin sulfate with a liquid chromatography- mass spectrometry.

[MEANS TO SOLVE THE PROBLEM]

In the research for the objective described above, the inventors have conducted intensive studies, and thus, have found that chondroitin sulfate configuring GAG (in particular, chondroitin 4-sulfate) can be efficiently decomposed into disaccharides by decomposing the chondroitin sulfate in the presence of HCl-methanol, and the amount of chondroitin sulfate can be sensitively measured by analyzing the disaccharides obtained by the decomposition with a liquid chromatography-mass spectrometry, and have completed the invention. Thus the invention includes the followings.

1. A method for decomposing chondroitin sulfate contained in a sample into disaccharide represented by formula (IV) below:

(Chem. 4)

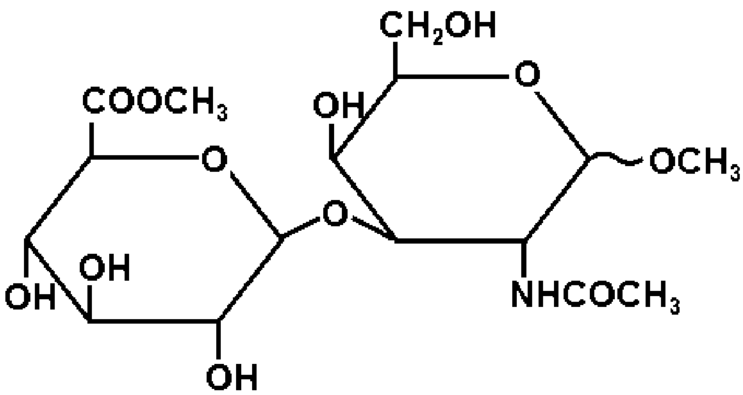

(IV)

wherein the chondroitin sulfate is decomposed by heating in HCl-methanol containing 2,2-dimethoxypropane at a temperature of 60°C to 90°C for 50 minutes to 180 minutes.

2. The method according to 1 above, wherein the heating for decomposing the chondroitin sulfate is performed at a temperature of 65°C to 75°C for 70 minutes to 110 minutes.

3. The method according to 1 or 2 above, wherein the sample is selected from a body fluid, a cell, a tissue, an organ, a cell culture medium, a tissue culture medium, a food, and a feed, or a derivative thereof.

4. The method according to 1 or 2 above, wherein the sample is selected from the group consisting of a body fluid, a cell, a tissue, an organ, blood, a blood serum, a blood plasma, urine, a bone marrow fluid, a cerebral spinal fluid, and a derivative thereof obtained from a mammal.

5. The method according to 4 above, wherein the mammal is selected from the group consisting of a human, a monkey, a mouse, a rat, a guinea pig, a hamster, a rabbit, a horse, a cow, a pig, a dog, and a cat.

6. The method according to 4 above, wherein the mammal is a human, and the human is a patient with a disorder in which chondroitin sulfate is accumulated in the body.

7. The method according to 6 above, wherein the disorder is selected from the group consisting of Maroteaux-Lamy syndrome, Morquio syndrome type A, Morquio syndrome type B, and Sly syndrome.

8. The method according to 6 or 7 above, wherein the patient has been treated to reduce the chondroitin sulfate present in the body.

9. A method for decomposing chondroitin sulfate and heparan sulfate contained in a sample, wherein the chondroitin sulfate is decomposed into disaccharide represented by formula (IV) below, and

(Chem. 4)

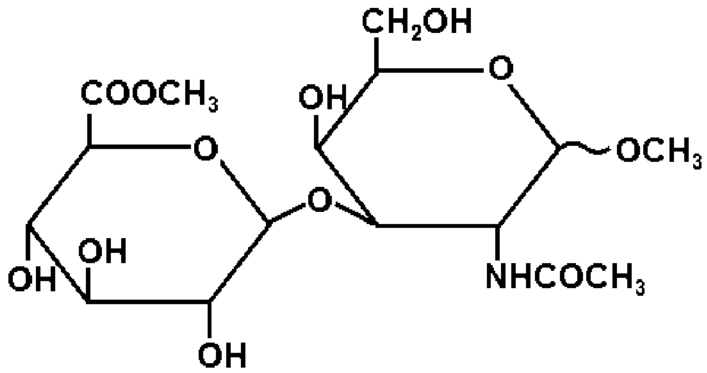

(IV)

the heparan sulfate is decomposed into disaccharide represented by formula (XIV) below, and

(Chem. 14)

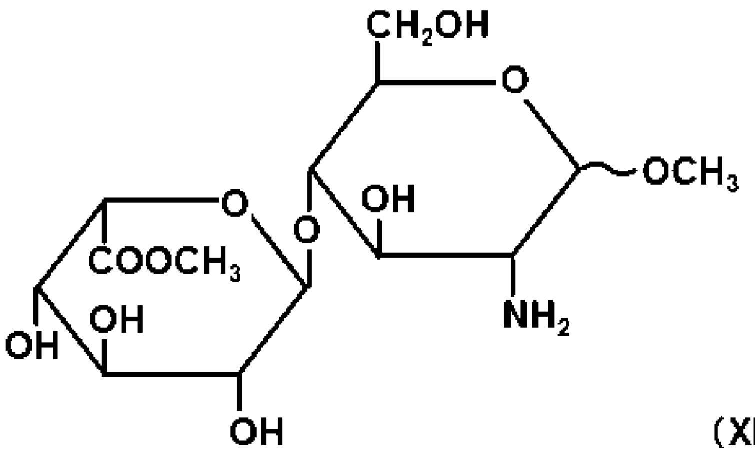

(XIV)

wherein the heparan sulfate is decomposed by heating in HCl-methanol containing 2,2-dimethoxypropane at a temperature of 65°C to 85°C for 80 minutes to 180 minutes, and the chondroitin sulfate is decomposed by the method according to 1 or 2 above.

10. The method according to 9 above, wherein the heating for decomposing the heparan sulfate is performed at a temperature of 78°C to 82°C for 110 minutes to 130 minutes.

11. A method for decomposing chondroitin sulfate, heparan sulfate, and dermatan sulfate contained in a sample, wherein the chondroitin sulfate is decomposed into disaccharide represented by formula (IV) below,

(Chem. 4)

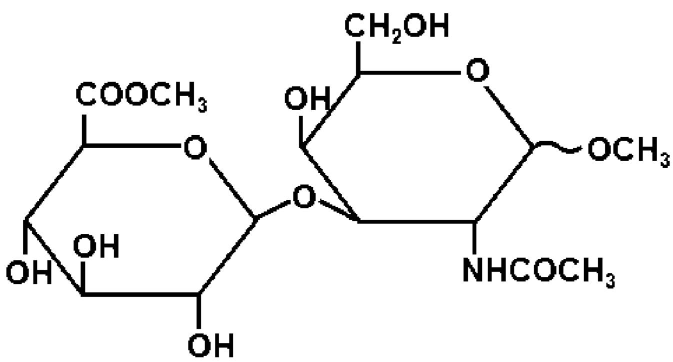

(IV)

the heparan sulfate is decomposed into disaccharide represented by formula (XIV) below, and

(Chem. 14)

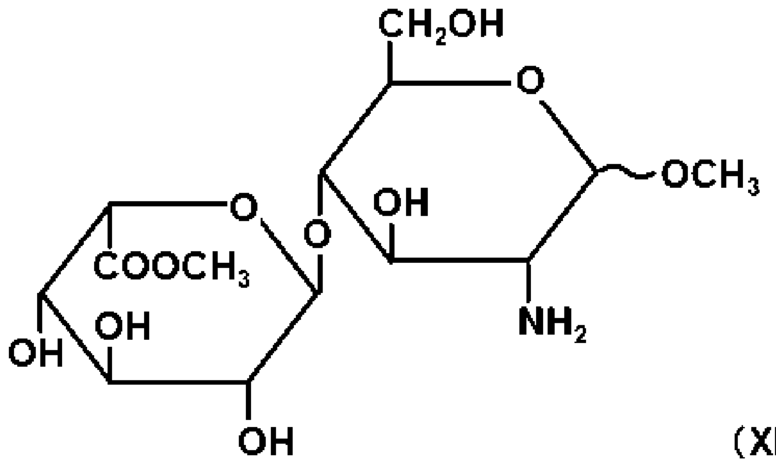

(XIV)

the dermatan sulfate is decomposed into disaccharide represented by formula (XII) below, and

(Chem. 12)

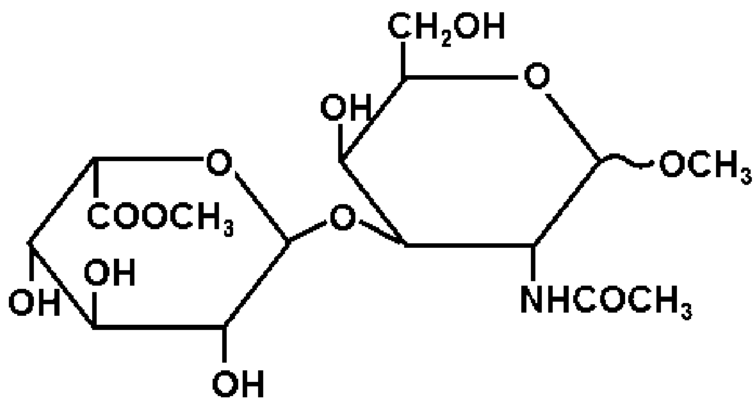

(XII)

wherein the dermatan sulfate is decomposed by heating in HCl-methanol containing 2,2-dimethoxypropane at a temperature of 60°C to 80°C for 20 minutes to 100 minutes, and the chondroitin sulfate and the heparan sulfate are decomposed by the method according to 9 or 10 above.

12. The method according to 11 above, wherein the heating for decomposing the dermatan sulfate is performed at a temperature of 63°C to 67°C for 30 minutes to 80 minutes.

13. The method according to any one of 9 to 12 above, wherein the sample is selected from a body fluid, a cell, a tissue, an organ, a cell culture medium, a tissue culture medium, a food, a feed, and the derivative thereof.

14. The method according to any one of 9 to 12 above, wherein the sample is selected from the group consisting of a body fluid, a cell, a tissue, an organ, a blood, a blood serum, a blood plasma, a urine, a bone marrow fluid, a cerebral spinal, and the derivative thereof obtained from a mammal.

15. The method according to 14 above, wherein the mammal is selected from the group consisting of a human, a monkey, a mouse, a rat, a guinea pig, a hamster, a rabbit, a horse, a cow, a pig, a dog, and a cat.

16. The method according to 14 above, wherein the mammal is a human, and the human is a patient with a disorder in which one or a plurality of chondroitin sulfate, heparan sulfate, and dermatan sulfate have been accumulated in the body.

17. The method according to 16 above, wherein the disorder is selected from the group consisting of Maroteaux-Lamy syndrome, Morquio syndrome type A, Morquio syndrome type B, Hunter syndrome, Hurler syndrome, Scheie syndrome, Hurler-Scheie syndrome, Sanfilippo syndrome, and Sly syndrome.

18. The method according to 16 or 17 above, wherein the patient has been treated to reduce one or a plurality of chondroitin sulfate, heparan sulfate, and dermatan sulfate present in the body.

19. A method for measuring the amount of chondroitin sulfate contained in a sample, the method including:

a step of obtaining an eluate by applying disaccharide obtained by the method according to any one of 1 to 8 above to liquid chromatography; and
a step of applying the eluate to mass spectrometry.

20. A method for measuring the amount of chondroitin sulfate and heparan sulfate contained in a sample, the method including:

a step of obtaining an eluate by applying disaccharide obtained by decomposing chondroitin sulfate and heparan sulfate by the method according to 9 or 10 above to liquid chromatography; and
a step of applying the eluate to mass spectrometry.

21. A method for measuring the amount of chondroitin sulfate, heparan sulfate, and dermatan sulfate contained in a sample, the method including:

a step of obtaining an eluate by applying disaccharide obtained by decomposing chondroitin sulfate, heparan sulfate, and dermatan sulfate by the method according to any one of 10 to 18 above to liquid chromatography; and
a step of applying the eluate to mass spectrometry.

22. A method for detecting an individual with a disorder in which chondroitin sulfate is accumulated in the body from among a mammal providing a sample, the sample obtained by the method according to 19 above, on the basis of a measurement value of chondroitin sulfate contained in the sample.
23. A method for detecting an individual with a disorder in which chondroitin sulfate and/or heparan sulfate is accumulated in the body from among a mammal providing a sample, the sample obtained by the method according to 20 above, on the basis of a measurement value of chondroitin sulfate and/or heparan sulfate contained in the sample.
24. A method for detecting an individual with a disorder in which one or a plurality of chondroitin sulfate, heparan sulfate, and dermatan sulfate are accumulated in the body from a mammal providing a sample, the sample obtained by the method according to 21 above, on the basis of measurement values of chondroitin sulfate, heparan sulfate, and dermatan sulfate.
25. The method according to any one of 22 to 24 above, wherein the disorder is selected from the group consisting of Maroteaux-Lamy syndrome, Morquio syndrome type A, Morquio syndrome type B, Hunter syndrome, Hurler syndrome, Scheie syndrome, Hurler-Scheie syndrome, Sanfilippo syndrome, and Sly syndrome.
26. A method for confirming an effect of a medical treatment, on the basis of a measurement value obtained by measuring the amount of a substance accumulated in a body and contained in samples by the method according to any one of 19 to 21 above,
wherein the samples are obtained from a patient, the patient suffering from a disorder in which one or a plurality of chondroitin sulfate are accumulated in the body and receiving a medical treatment to reduce the amount of the substance in the body, before and after receiving the treatment.

[EFFECT OF INVENTION]

According to the present invention, for example, the amount of chondroitin sulfate contained in a sample (blood serum, cerebral spinal fluid, and the like) collected from a mammal can be sensitively measured independently or along with heparan sulfate, or along with dermatan sulfate in addition thereto.

[BRIEF DESCRIPTION OF DRAWINGS]

Fig. 1 is an ion chromatogram obtained by analyzing a solution for preparing a standard curve, the solution containing 250 ng/mL of chondroitin sulfate and subjected to methanolysis, by LC/MS/MS analysis. The vertical axis indicates counts per second (cps), and the horizontal axis indicates an elution time (minute).
Fig. 2 is a graph showing a standard curve of chondroitin sulfate. The vertical axis indicates an area ratio (CS Detection Peak Area/DS-IS Detection Peak Area), and the horizontal axis indicates a concentration (ng/mL) of chondroitin sulfate.
Fig. 3 is an ion chromatogram obtained by analyzing a solution for preparing a standard curve, the solution containing

25 ng/mL of dermatan sulfate and 25 ng/mL of heparan sulfate and subjected to methanolysis in a condition A shown in Table 4, by LC/MS/MS analysis. The vertical axis indicates counts per second (cps), and the horizontal axis indicates an elution time (minute).

Fig. 4 is an ion chromatogram obtained by analyzing a solution for preparing a standard curve, the solution containing 25 ng/mL of dermatan sulfate and 25 ng/mL of heparan sulfate and subjected to methanolysis in a condition B shown in Table 4, by LC/MS/MS analysis. The vertical axis indicates counts per second (cps), and the horizontal axis indicates an elution time (minute).

Fig. 5 is a graph showing measurement results of a concentration of dermatan sulfate contained in blood serum of a mouse. The vertical axis indicates a concentration (μg/mL) of dermatan sulfate in mouse blood serum, a white bar indicates a concentration of dermatan sulfate in blood serum of a wild type mouse, a black bar indicates a concentration of dermatan sulfate in blood serum of an IDS hemizygote mouse, an error bar indicates a standard deviation (n = 3), (A) indicates results obtained by measuring the concentration of the dermatan sulfate subjected to methanolysis at 65°C for 75 minutes, (B) indicates results obtained by measuring the concentration of the dermatan sulfate subjected to methanolysis at 80°C for 2 hours, and (C) indicates results obtained by measuring the concentration of the dermatan sulfate subjected to methanolysis at 65°C for 18 hours.

Fig. 6 is a graph showing the results of measuring a concentration of heparan sulfate contained in blood serum of a mouse. The vertical axis indicates a concentration (μg/mL) of heparan sulfate in mouse blood serum, a white bar indicates results of a concentration of heparan sulfate in blood serum of a wild type mouse, a black bar indicates results of a concentration of heparan sulfate in blood serum of an IDS hemizygote mouse, an error bar indicates a standard deviation (n=3), (A) indicates results obtained by measuring the concentration of the heparan sulfate subjected to methanolysis at 65°C for 75 minutes, (B) indicates results obtained by measuring the concentration of the heparan sulfate subjected to methanolysis at 80°C for 2 hours, and (C) indicates results obtained by measuring the concentration of the heparan sulfate subjected to methanolysis at 65°C for 18 hours.

Fig. 7 is a graph showing measurement results of a concentration of heparan sulfate and dermatan sulfate contained in blood serum of a rhIDS-treated mouse. The vertical axis indicates a concentration (μg/mL) of heparan sulfate and dermatan sulfate in mouse blood serum, a white bar indicates results of a wild type mouse, a black bar indicates results of a rhIDS-untreated IDS hemizygote mouse, a shaded bar indicates results of a rhIDS-treated IDS hemizygote mouse, an error bar indicates a standard deviation, a double # sign indicates $p < 0.01$ in a t-test (a comparison between the wild type mouse and the rhIDS-untreated IDS hemizygote mouse), a double star sign indicates $p < 0.01$ in a t-test (a comparison between the rhIDS-untreated IDS hemizygote mouse and the rhIDS-untreated IDS hemizygote mouse), (A) indicates measurement results of the concentration of the heparan sulfate, and (B) indicates measurement results of the concentration of the dermatan sulfate.

## DETAILED DESCRIPTION OF THE INVENTION

In the present invention, "chondroitin sulfate" generally means a molecule having a repeating structure of disaccharide in which a D-glucuronic acid (GlcA) and N-acetyl-D-galactosamine (GalNAc) are β1,3-bonded wherein the molecule is modified with a sulfate group. A molecule having a repeating structure of disaccharide of GlcA and GalNAc having a sulfate group bonded at a position 4 is denoted as chondroitin 4-sulfate. A molecule having a repeating structure of disaccharide of GlcA and GalNAc having a sulfate group bonded at a position 6 is denoted as chondroitin 6-sulfate. In addition, the chondroitin sulfate includes molecular species such as chondroitin sulfate D, chondroitin sulfate E, and chondroitin sulfate K. The disaccharide that is the unit of the repeating structure of the chondroitin sulfate, for example, is represented by General Formula (I) described below.

[Chem. 1]

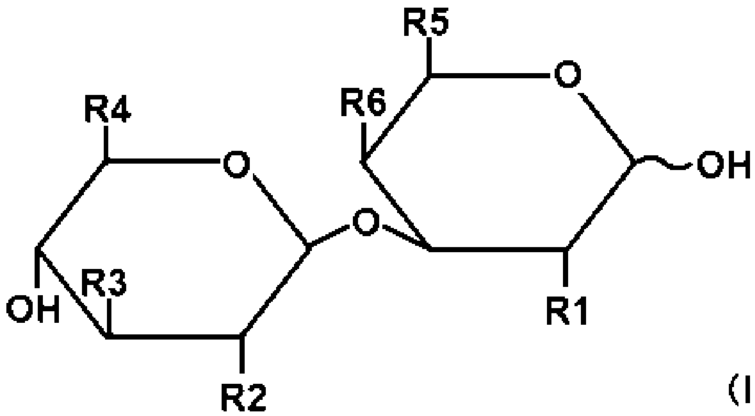

(I)

[In Formula (I), $R_1$ is $NH_2$, $NHCOCH_3$, $NHSO_3H$, or a salt thereof; $R_2$ is OH, $OSO_3H$, or a salt thereof, $R_3$ is OH, $OSO_3H$, or a salt thereof; $R_4$ is COOH or a salt thereof; $R_5$ is $CH_2OH$, $CH_2OSO_3H$, or a salt thereof; $R_6$ is OH, $OSO_3H$, or a salt thereof. Here, any one of $R_1$, $R_2$, $R_3$, $R_4$, and $R_5$ has a sulfate group.

In particular, a molecule in which in Formula (I), $R_1$ is $NHCOCH_3$ or a salt thereof; $R_2$ is OH; $R_3$ is OH; $R_4$ is COOH or a salt thereof; $R_5$ is $CH_2OH$; and $R_6$ is $OSO_3H$ or a salt thereof is chondroitin 4-sulfate.

In addition, a molecule in which in Formula (I), $R_1$ is $NHCOCH_3$ or a salt thereof; $R_2$ is OH; $R_3$ is OH; $R_4$ is COOH or a salt thereof; $R_5$ is $CH_2OSO_3H$ or a salt thereof; and $R_6$ is OH is chondroitin 6-sulfate.

In addition, a molecule in which in Formula (I), $R_1$ is $NHCOCH_3$ or a salt thereof; $R_2$ is $OSO_3H$ or a salt thereof; $R_3$ is OH; $R_4$ is COOH or a salt thereof; $R_5$ is $CH_2OSO_3H$ or a salt thereof; and $R_6$ is OH is chondroitin sulfate D.

In addition, a molecule in which in Formula (I), $R_1$ is $NHCOCH_3$ or a salt thereof; $R_2$ is OH; $R_3$ is OH; $R_4$ is COOH or a salt thereof; $R_5$ is $CH_2OSO_3H$ or a salt thereof; and $R_6$ is $OSO_3H$ or a salt thereof is chondroitin sulfate E.

In addition, a molecule in which in Formula (I), $R_1$ is $NHCOCH_3$ or a salt thereof; $R_2$ is OH; $R_3$ is $OSO_3H$ or a salt thereof; $R_4$ is COOH or a salt thereof; $R_5$ is $CH_2OH$; and $R_6$ is $OSO_3H$ or a salt thereof is chondroitin sulfate K.]

Methanolysis (methanol decomposition) means a reaction for decomposing the chondroitin sulfate into disaccharides in which a D-glucuronic acid having a methylated carboxyl group and N-acetyl-D-galactosamine having a methylated hydroxyl group at a position 1 are β1,3-bonded, in HCl-methanol. A reaction equation of the methanolysis of the chondroitin 4-sulfate having the repeating structure of the disaccharide of GlcA and GalNAc having a sulfate group bonded at a position 4 is represented by Formula (II) described below, as an example.

[Chem. 2]

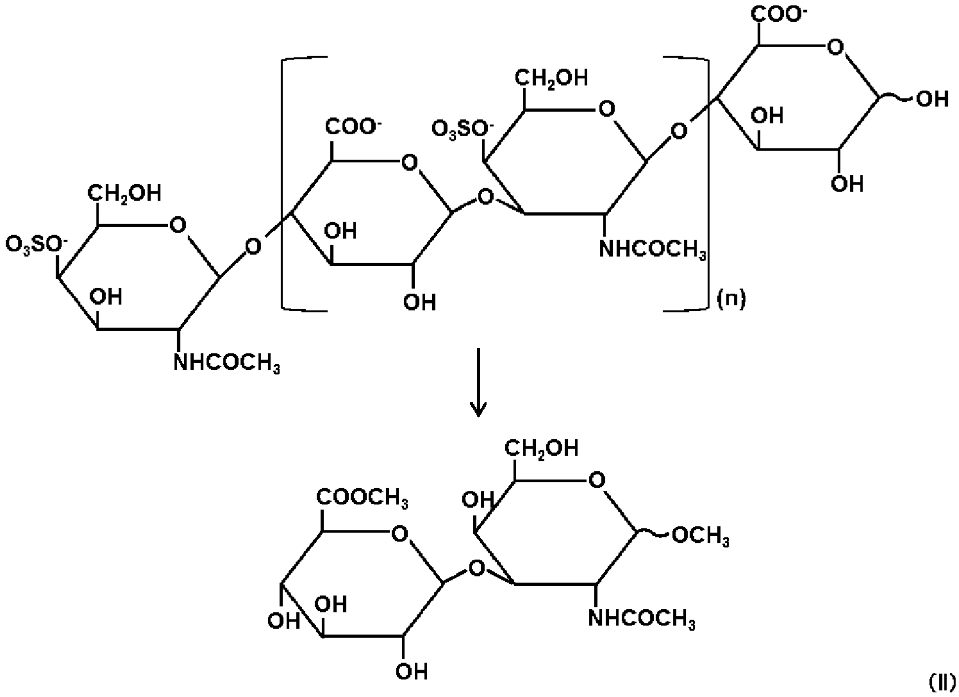

(II)

[In Formula (II), n represents an integer which is greater than or equal to 1.]

In addition, a reaction equation of the methanolysis of the chondroitin 6-sulfate having the repeating structure of the disaccharide of GlcA and GalNAc having a sulfate group bonded at a position 6 is represented by Formula (III) described below, as an example.

[Chem. 3]

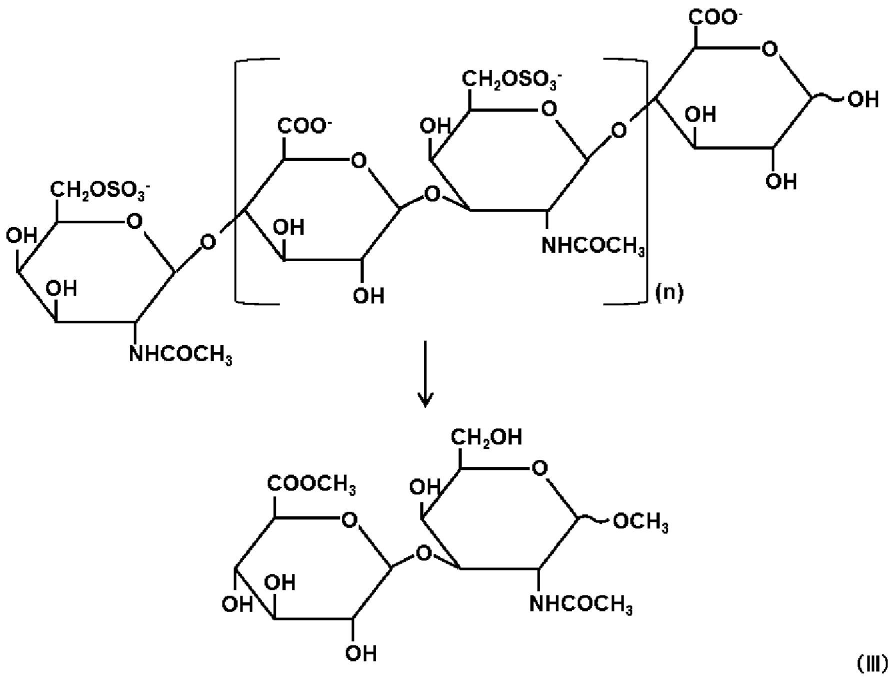

(III)

[In Formula (III), n represents an integer which is greater than or equal to 1.]

As represented by Formula (II) and Formula (III), the disaccharide generated by the methanolysis is the same

in the chondroitin 4-sulfate and the chondroitin 6-sulfate, and is represented by Formula (IV) described below.

[Chem. 4]

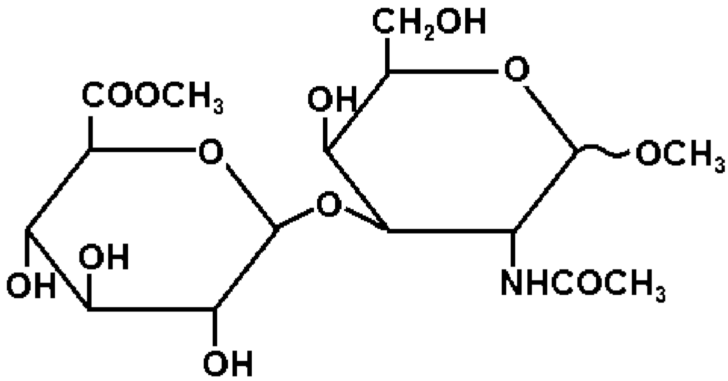

(IV)

A methanol decomposition reaction (the methanolysis) of the chondroitin sulfate is performed by heating a sample containing the chondroitin sulfate in HCl-methanol containing 2,2-dimethoxypropane. Here, the HCl-methanol means methanol containing hydrogen chloride. The concentration of the hydrogen chloride contained in the HCl-methanol is preferably 0.5 to 5 mol/L, is more preferably 1 to 4 mol/L, and is even more preferably 2.5 to 3.5 mol/L, and for example, is 3 mol/L. Here, a ratio of the 2,2-dimethoxypropane to the HCl-methanol is not particularly limited, and for example, is 0.5 to 1.5 : 10 (v/v), and is particularly 1 : 10 (v/v). In addition, in this case, a heating condition in the methanol decomposition reaction is preferably a temperature of 60°C to 90°C for 50 minutes to 180 minutes, is more preferably a temperature of 65°C to 75°C for 70 minutes to 110 minutes, and is even more preferably a temperature of 67°C to 73°C for 80 minutes to 100 minutes or 85 to 95 minutes, and for example, is a temperature of 70°C for 90 minutes.

The amount of chondroitin sulfate contained in the sample can be measured with a method of the present invention. The sample subjected to measuring chondroitin sulfate is heated in the condition of the methanol decomposition reaction described above. The chondroitin sulfate contained in the sample is decomposed into the disaccharide by the reaction.

The disaccharide obtained by decomposing the chondroitin sulfate with the methanol decomposition reaction (the methanolysis) is applied to liquid chromatography. Then, an eluate from the liquid chromatography is sequentially applied to mass analysis.

In this case, the liquid chromatography to be used is not particularly limited insofar as the disaccharide represented by Chemical Formula (IV) described above can be separated from other substances by being once adsorbed in a column, and then, by being eluted.

For example, high-performance liquid column chromatography using a column filled with carriers capable of adsorbing the disaccharide by an ionic interaction, a hydrophobic interaction, a hydrophilic interaction, and the like can be preferably used in the method of the present invention.

The disaccharide represented by Formula (IV) that is generated by the methanol decomposition reaction of the chondroitin sulfate exemplified by chemical reaction equations (II) and (III) described above has high polarity. Accordingly, a carrier capable of retaining disaccharide by a hydrophilic interaction is particularly preferable as the carrier capable of once adsorbing the disaccharide, and then, of eluting the disaccharide. That is, high-performance liquid chromatography using the carrier capable of retaining the disaccharide by the hydrophilic interaction is preferable as a method for separating the disaccharide obtained with the methanol decomposition reaction. Examples of the high-performance liquid chromatography include hydrophilic interaction liquid chromatography used in Example 4 as described below.

A flow path from an outlet of the liquid chromatography is connected to a mass spectrometer, and the eluate from the liquid chromatography is sequentially sent to the mass analysis.

In this case, the mass spectrometer to be used is not particularly limited. For example, in the mass spectrometer, any ionization method including a photoionization method (APPI), an electronic ionization method (EI), a chemical ionization method, an electron desorption method, a fast atom bombardment method (FAB), a matrix-assisted laser desorption/ionization method (MALDI), and an electrospray ionization method (ESI) may be adopted as an ion source for ionizing molecules that are an analysis target. In addition, in the mass spectrometer, an analysis unit for separating ionized molecules may be any type including a magnetic field deflection type, a quadrupole type, an ion trap type, and a tandem quadrupole type.

A mass spectrometer including an ion source operated in an electrospray ionization method (ESI) that is operated in a cation mode and a tandem quadrupole type analysis unit can be preferably used in the method of the

present invention. A tandem quadrupole type mass spectrometer is a mass spectrometer in which a quadrupole (Q1) functioning as a mass filter, a quadrupole (Q2) functioning as a collision cell, and a quadrupole (Q3) functioning as a mass filter are arranged in series. In the quadrupole (Q1), target precursor ions are separated from a plurality of ions generated by ionization, on the basis of a mass charge ratio (m/z) of the ions. Next, in the collision cell (Q2), the precursor ions collide with inactive gas (for example, argon) or the like, and thus, product ions (fragment ions) are generated. Next, in the quadrupole (Q3), the obtained product ions are selectively detected on the basis of the mass charge ratio (m/z).

Hereinafter, it will be exemplified that a specific example of a method for generating the precursor ions and the product ions from the disaccharide represented by Formula (IV) obtained by the methanol decomposition of the chondroitin 4-sulfate represented by Formula (II) described above or the chondroitin 6-sulfate represented by Formula (III) described above, by the tandem quadrupole type mass spectrometer. First, precursor ions having a mass charge ratio (m/z) of 426 can be obtained by ionizing the disaccharide. The precursor ions are represented by Formula (V) described below.

[Chem. 5]

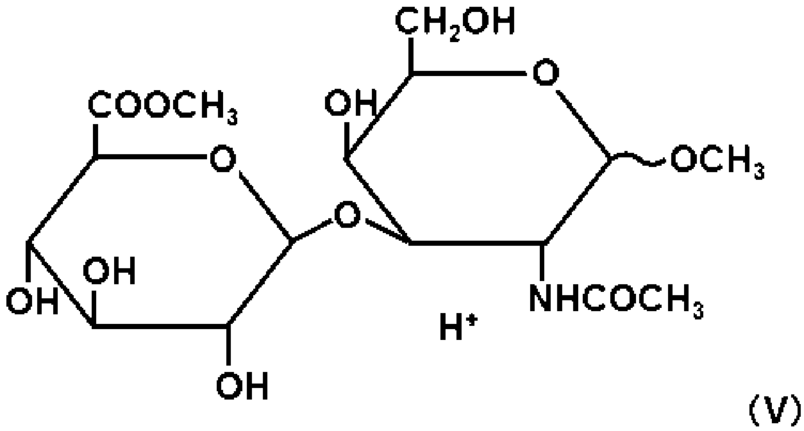

(V)

The precursor ions represented by Formula (V) described above are separated, and are cleaved in the collision cell (Q2), and thus, product ions having a mass charge ratio (m/z) of 236 can be obtained. A reaction for obtaining the product ions by cleaving the precursor ions is schematically represented by Formula (VI).

[Chem. 6]

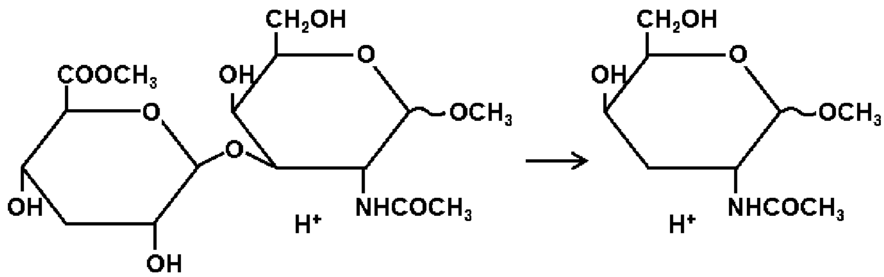

(VI)

The amount of chondroitin sulfate contained in the sample can be measured by a method using the liquid chromatography and the tandem quadrupole type mass spectrometer described above. A known amount of chondroitin sulfate is subjected to the methanol decomposition and is analyzed by using the tandem quadrupole type mass spectrometer, and thus, the area of a peak (a detection peak) found on a chromatography chart corresponding to product ions having a mass charge ratio (m/z) of 236 is calculated. Then, a standard curve representing a correlation between the amount of chondroitin sulfate and the area of the detection peak is prepared. In addition, likewise, a sample in which the content of chondroitin sulfate is unknown is subjected to the methanol decomposition and is analyzed by using the tandem quadrupole type mass spectrometer, and thus, the area of the detection peak corresponding to the product ions is calculated. The obtained value is interpolated into the standard curve, and thus, the chondroitin sulfate contained in the sample can be measured.

In the present invention, the sample that is an analysis target is not particularly limited, and examples thereof include body fluid, a cell, a tissue, an organ, a cell culture solution, a tissue culture solution, food, and feed, or a derivative

therefrom.

In a case where the sample is a cell, organism species from which the cell is derived is not particularly limited. The organism species may be a procaryote or a eucaryote, and for example, is a bacterium, a yeast, a plant, a bird, an amphibian, a reptile, and a mammal. In a case where the organism species is a mammal, the animal species are not particularly limited, and for example, are a human, a monkey, a mouse, a rat, a guinea pig, a hamster, a rabbit, a horse, a cow, a pig, a dog, and a cat, and in particular, the animal species are a human.

In a case where the sample is body fluid, a tissue, or an organ, organism species from which the body fluid, the tissue, or the organ is derived are not particularly limited. Examples of the organism species include a plant, a bird, an amphibian, a reptile, and a mammal, and in particular, the organism species are a mammal. In a case where the organism species are a mammal, animal species are not particularly limited, and for example, are a human, a monkey, a mouse, a rat, a guinea pig, a hamster, a rabbit, a horse, a cow, a pig, a dog, and a cat, and in particular, the animal species are a human.

In a case where the sample is derived from a mammal, the sample, for example, is body fluid, blood, blood serum, blood plasma, bone marrow fluid, cerebral spinal fluid, and urine obtained from the mammal, or is derived therefrom. Here, the body fluid means a liquid component generally derived from a mammal, including blood, bone marrow fluid, cerebral spinal fluid, saliva, tears, sweat, semen, synovial fluid, and urine. The blood serum or the blood plasma can be prepared from the blood by a known method such as centrifugal separation.

In addition, in a case where the sample is derived from a mammal, the sample, for example, is a cell, a tissue, and an organ obtained from the mammal, or is derived therefrom. Here, the type of cell is not particularly limited, and examples of the cell include a mesenchymal stem cell, a nerve cell, a neuroblast, a myoblast cell, a neuroglial cell, a Schwann cell, a cardiac muscle cell, a skeletal muscle cell, a smooth muscle cell, a cartilage cell, an osteoblast cell, a fibroblast cell, a keratinocyte, an epidermal cell, an endothelial cell, a corneal epidermal cell, a corneal endothelial cell, a retina cell, a liver cell, a mesangial cell, a mesenchymal cell, a blood cell, a blood-forming cell, a dendritic cell, and an interstitial cell. In addition, the tissue is not also particularly limited, and examples of the tissue include an epidermal tissue, a connective tissue, a muscle tissue, a nerve tissue, a fibrous connective tissue, a cartilage tissue, and a bone tissue. In addition, the organ is not also particularly limited, and examples of the organ include a stomach, a small intestine, a large intestine, a liver, a pancreas, a kidney, a spleen, a heart, a lung, a pituitary, a testicle, an ovary, a cerebellum, a cerebrum, an interbrain, a midbrain, medulla oblongata, and a hypophysis.

A disorder in which chondroitin sulfate is accumulated in the body have been known. For example, such disorder includes a Maroteaux-Lamy syndrome, a Morquio syndrome type A, a Morquio syndrome type B, and a Sly syndrome. In such a disorder, a method of treatment for alleviating a symptom by reducing the chondroitin sulfate abnormally accumulated in the body has been tested. An enzyme replacement therapy for dosing the body with an enzyme for decomposing the chondroitin sulfate is an example of such a method of treatment.

A screening for a patient with the disorder in which the chondroitin sulfate is accumulated in the body can be performed on the basis of a measurement value that is obtained by measuring the concentration of chondroitin sulfate contained in the blood. In a case where the measurement value of a subject is abnormally higher than that of a normal human, it can be determined that the subject is a patient with the disorder. The method of the present invention can be implemented in order to perform such determination.

When the patient with the disorder in which the chondroitin sulfate is accumulated in the body is subjected to a medical treatment, the effect of the medical treatment can be checked by measuring the chondroitin sulfate accumulated in the body of the patient before and after the medical treatment, and by measuring a reduction amount after the medical treatment. It can be determined that the larger the reduction amount is, the higher the effect of the medical treatment is. The method of the present invention can be implemented in order to check an effect of such a medical treatment. For example, it can be determined that the effect of the medical treatment is obtained in a case where the concentration of the chondroitin sulfate contained in the blood of the patient is reduced by preferably greater than or equal to 10%, more preferably greater than or equal to 20%, even more preferably greater than or equal to 30%, and still even more preferably greater than or equal to 50%, before and after the medical treatment, respectively.

In addition, the screening of a medical agent for the medical treatment of the patient with the disorder in which the chondroitin sulfate is accumulated in the body can be performed by dosing an experimental animal with a test drug, by measuring the chondroitin sulfate accumulated in the body of the experimental animal before and after the dose of the test drug, and by measuring a reduction amount after the dose. It can be determined that the larger the reduction amount is, the higher the effect of the test drug is. For example, it can be determined that the effect of the medical treatment of the test drug increases in a case where the concentration of the chondroitin sulfate contained in the blood of the patient is reduced by preferably greater than or equal to 10%, more preferably greater than or equal to 20%, even more preferably greater than or equal to 30%, and still even more preferably greater than or equal to 50%, before and after the medical treatment, respectively.

The Maroteaux-Lamy syndrome is a disorder in which a part or all of N-acetyl galactosamine-4-sulfate sulfatase (ASB) activity is genetically lacked. The chondroitin sulfate is accumulated in the body of the patient by the defect or

deletion of ASB. Accordingly, the method of the present invention can be used in the screening of a patient with the Maroteaux-Lamy syndrome, the check of the effect of a method of treatment thereof, the evaluation of a medicinal effect of a therapeutic agent, the screening of a test drug thereof, and the like. Examples of the therapeutic agent of the Maroteaux-Lamy syndrome include ASB, an analog of ASB, and a bonded body of ASB and an antibody, but the therapeutic agent is not limited thereto. Examples of the bonded body of ASB and the antibody include fusion protein of ASB and an anti-human transferrin receptor antibody.

In addition, the Morquio syndrome type A is a disorder in which a part or all of N-acetyl galactosamine-6-sulfate sulfatase activity is genetically lacked, and the Morquio syndrome type B is a disorder in which a part or all of β-galactosidase activity is genetically lacked. The chondroitin sulfate is accumulated in the body of the patient by the defect or deletion of the enzyme. Accordingly, the method of the present invention can be used in the screening of a patient with the Morquio syndrome type A and a Morquio syndrome type B, the check of the effect of a method of treatment thereof, the evaluation of a medicinal effect of a therapeutic agent, the screening of a test drug thereof, and the like. Examples of the therapeutic agent of the Morquio syndrome type A include N-acetyl galactosamine-6-sulfate sulfatase, an analog thereof, and a bonded body of an enzyme and an antibody, but the therapeutic agent is not limited thereto. Examples of the bonded body of the enzyme and the antibody include fusion protein of an enzyme and an anti-human transferrin receptor antibody. Examples of the therapeutic agent of the Morquio syndrome type B include β-galactosidase, an analog thereof, and a bonded body of an enzyme and an antibody, but the therapeutic agent is not limited thereto. Examples of the bonded body of the enzyme and the antibody include fusion protein of an enzyme and an anti-human transferrin receptor antibody.

In addition, the Sly syndrome is a disorder in which a part or all of β-glucuronidase activity is genetically lacked. The chondroitin sulfate is accumulated in the body of the patient by the defect or deletion of the β-glucuronidase. Accordingly, the method of the present invention can be used in the screening of a patient with the Sly syndrome, the check of the effect of a method of treatment thereof, the evaluation of a medicinal effect of a therapeutic agent thereof, the screening of a test drug thereof, and the like. Examples of the therapeutic agent of the Sly syndrome include β-glucuronidase, an analog thereof, and a bonded body of β-glucuronidase and an antibody, but the therapeutic agent is not limited thereto. Examples of the bonded body of the β-glucuronidase and the antibody include fusion protein of β-glucuronidase and an anti-human transferrin receptor antibody.

The method of the present invention can be used as a method for measuring the amount of chondroitin sulfate contained in food and feed. For example, the amount of chondroitin sulfate contained in food or feed ingested by a human or an animal other than the human is measured in advance, and thus, an ingestion amount of chondroitin sulfate can also be controlled. When a patient with the disorder described above is in a state in which the ingestion amount of chondroitin sulfate is to be limited, the ingestion amount of chondroitin sulfate can be suitably controlled by ingesting food in which the amount of chondroitin sulfate is measured in advance with the method of the present invention.

In the present invention, "dermatan sulfate" generally means a molecule having a repeating structure of disaccharide in which an uronic acid and amino sugar are α1,3-bonded. Examples of the uronic acid include L-iduronic acid and L-iduronic acid-2-sulfate, and examples of the amino sugar include N-acetyl-D-galactosamine-4-sulfate, but the uronic acid and the amino sugar are not limited thereto. The disaccharide that is the unit of the repeating structure of the dermatan sulfate, for example, is represented by General Formula (VII) described below.

[Chem. 7]

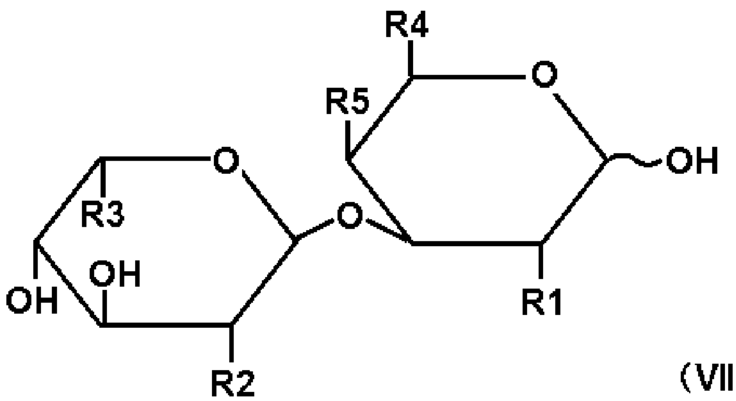

(VII)

[In Formula (VII), $R_1$ is $NH_2$, $NHCOCH_3$, $NHSO_3H$, or a salt thereof; $R_2$ is OH, $OSO_3H$, or a salt thereof, $R_3$ is COOH or a salt thereof; $R_4$ is $CH_2OH$,$CH_2OSO_3H$, or a salt thereof; and $R_5$ is OH, $OSO_3H$, or a salt thereof. Here, any

one of $R_1$, $R_2$, $R_4$, and $R_5$ has a sulfate group.]

A molecule represented by Formula (VIII) described below is a more specific example of the disaccharide that is the unit of the repeating structure of the dermatan sulfate, in which an uronic acid is an L-iduronic acid, and amino sugar is N-acetyl-D-galactosamine-4-sulfate.

[Chem. 8]

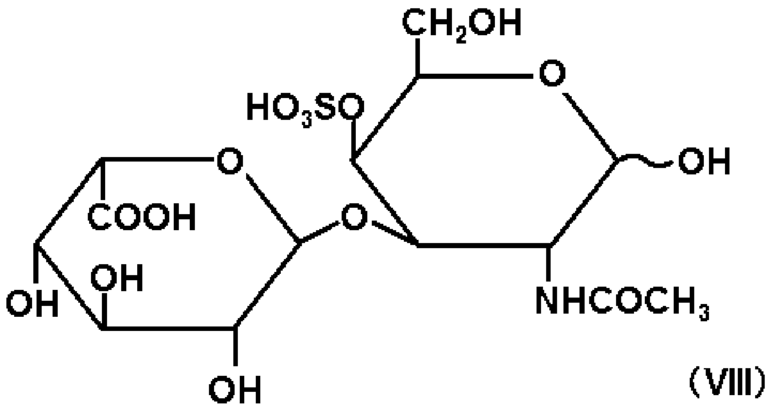

(VIII)

In the present invention, "heparan sulfate" generally means a molecule having a repeating structure of disaccharide in which an uronic acid and amino sugar are $\alpha$1,4-bonded. Examples of the uronic acid include an L-iduronic acid and L-iduronic acid-2-sulfate, and examples of the amino sugar include D-glucosamine and N-sulfo-D-glucosamine-6-sulfate, but the uronic acid and the amino sugar are not limited thereto. The disaccharide that is the unit of the repeating structure of the heparan sulfate, for example, is represented by General Formula (IX) described below.

[Chem. 9]

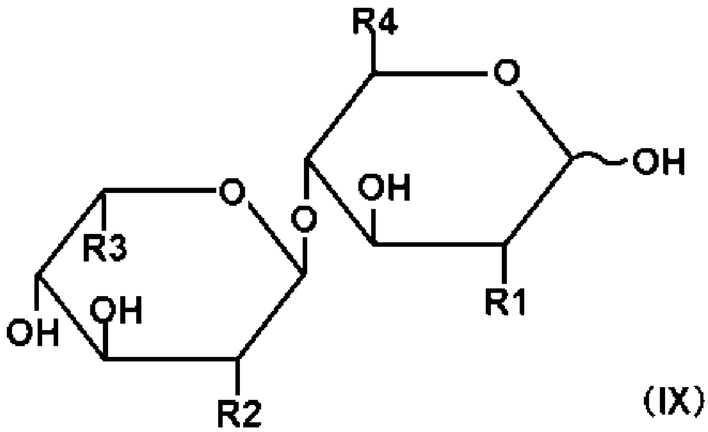

(IX)

[In Formula (IX), $R_1$ is $NH_2$, $NHCOCH_3$, $NHSO_3H$, or a salt thereof; $R_2$ is OH, $OSO_3H$, or a salt thereof; $R_3$ is COOH or a salt thereof; and $R_4$ is $CH_2OH$,$CH_2OSO_3H$, or a salt thereof. Here, any one of $R_1$, $R_2$, and $R_4$ has a sulfate group.]

A molecule represented by Formula (X) described below is a more specific example of the disaccharide that is the unit of the repeating structure of the heparan sulfate, in which an uronic acid is L-iduronic acid-2-sulfate, and amino sugar is N-sulfo-D-glucosamine-6-sulfate.

[Chem. 10]

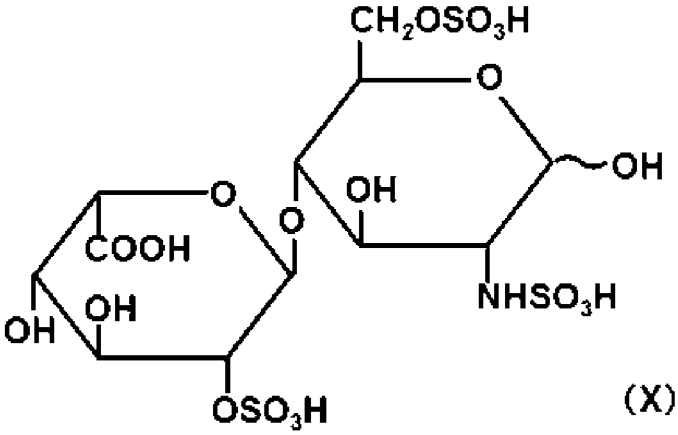

(X)

Methanolysis (methanol decomposition) is a reaction for decomposing the dermatan sulfate into a disaccharide in which an uronic acid having a methylated carboxyl group and an amino sugar having a methylated hydroxyl group at a position 1 are α1,3-bonded, in HCl-methanol. The same applies to a reaction for decomposing the heparan sulfate into a disaccharide in which an uronic acid having a methylated carboxyl group and an amino sugar having a methylated hydroxyl group at a position 1 are α1,4-bonded.

A reaction equation of the methanolysis of the dermatan sulfate in which the uronic acid is an L-iduronic acid, and the amino sugar is N-acetyl-D-galactosamine-4-sulfate is exemplified by Formula (XI) described below.

[Chem. 11]

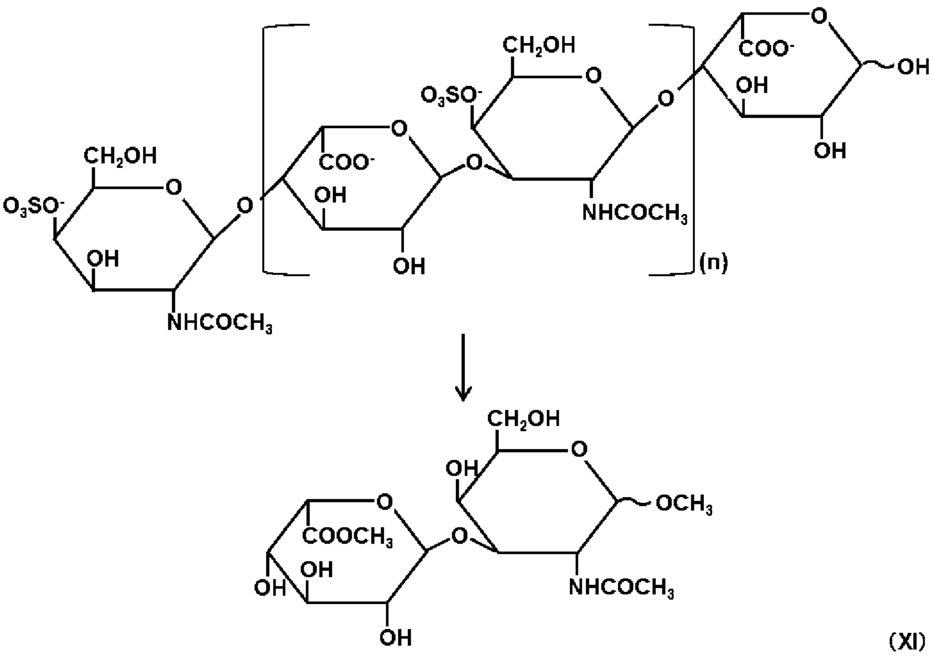

(XI)

[In Formula (XI), n represents an integer of greater than or equal to 1.]

The disaccharide generated by the methanolysis of the dermatan sulfate described above is represented by Formula (XII) described below.

[Chem. 12]

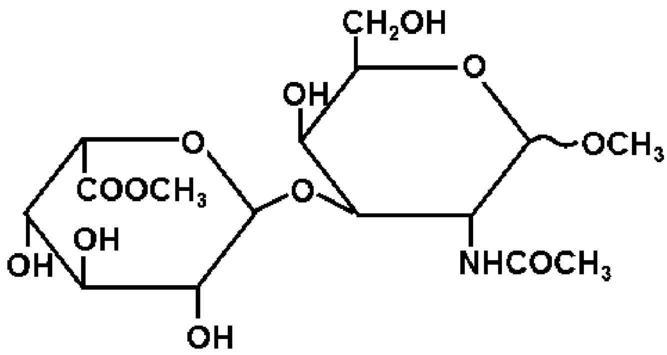

(XII)

A reaction equation of the methanolysis of the heparan sulfate in which the uronic acid is L-iduronic acid-2-sulfate, and the amino sugar is N-sulfo-D-glucosamine-6-sulfate is exemplified by Formula (XIII) described below.

[Chem. 13]

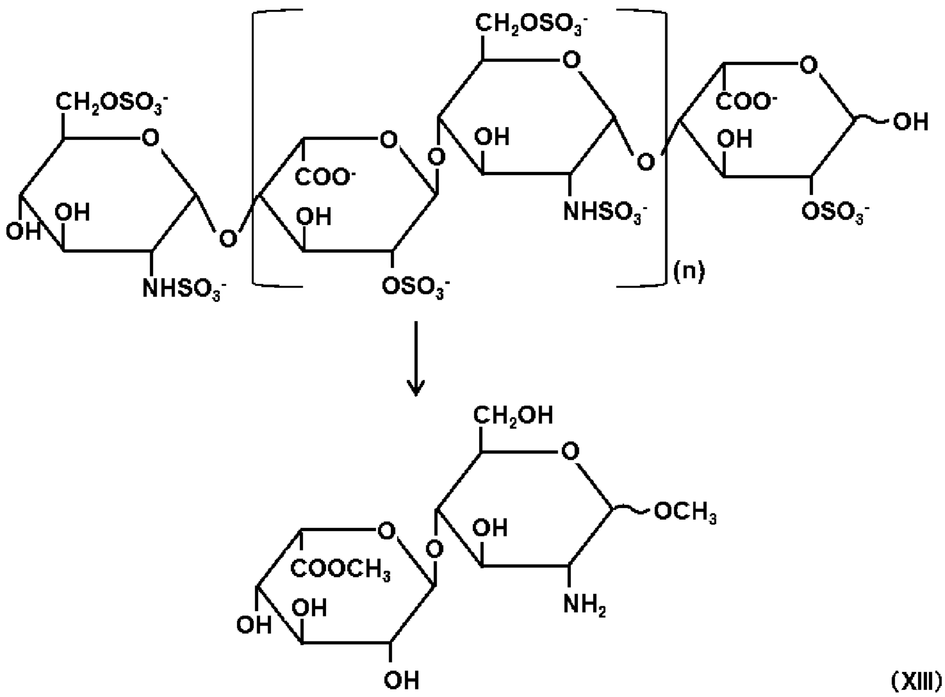

(XIII)

[In Formula (XIII), n represents an integer of greater than or equal to 1.]

The disaccharide generated by the methanolysis of the heparan sulfate described above is represented by Formula (XIV) described below.

[Chem. 14]

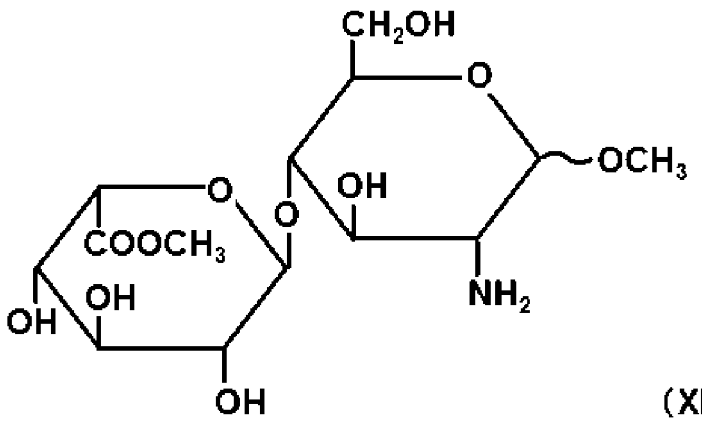

(XIV)

A methanol decomposition reaction (the methanolysis) of the dermatan sulfate is performed by heating a sample containing the dermatan sulfate in HCl-methanol containing 2,2-dimethoxypropane. Here, the HCl-methanol means methanol containing hydrogen chloride. The concentration of the hydrogen chloride contained in the HCl-methanol is preferably 0.5 to 5 mol/L, is more preferably 1 to 4 mol/L, and is even more preferably 2.5 to 3.5 mol/L, and for example, is 3 mol/L. Here, a ratio of the 2,2-dimethoxypropane to the HCl-methanol is not particularly limited, and for example, is 0.5 to 1.5 : 10, and is particularly 1 : 10. In addition, in this case, a heating condition in the methanol decomposition reaction is preferably a temperature of 60°C to 80°C for 20 minutes to 100 minutes, is more preferably a temperature of 60°C to 70°C for 20 minutes to 90 minutes, and is even more preferably a temperature of 63°C to 67°C for 30 minutes to 80 minutes or 60 to 80 minutes, and for example, is a temperature of 65°C for 75 minutes.

A methanol decomposition reaction (the methanolysis) of the heparan sulfate is performed by heating a sample containing the heparan sulfate in HCl-methanol containing 2,2-dimethoxypropane. Here, the concentration of the hydrogen chloride contained in the HCl-methanol is preferably 0.5 to 5 mol/L, is more preferably 1 to 4 mol/L, and is even more preferably 2.5 to 3.5 mol/L, and for example, is 3 mol/L. Here, a ratio of the 2,2-dimethoxypropane to the HCl-methanol is not particularly limited, and for example, is 0.5 to 1.5 : 10, and is particularly 1 : 10. In addition, in this case, a heating condition in the methanol decomposition reaction is preferably a temperature of 65°C to 85°C for 80 minutes to 180 minutes, is more preferably a temperature of 75°C to 85°C for 90 minutes to 180 minutes, and is even more preferably a temperature of 78°C to 82°C for 110 minutes to 130 minutes, and for example, is a temperature of 80°C for 120 minutes.

The amounts of dermatan sulfate and heparan sulfate contained in the sample can be measured with the method of the present invention. In this case, the sample to be measured is divided, one of the divided samples is used as a sample for measuring the dermatan sulfate, and the other one is used as a sample for measuring the heparan sulfate. The sample used for measuring the dermatan sulfate is heated in the condition of the methanol decomposition reaction of the dermatan sulfate described above. According to such a reaction, the dermatan sulfate contained in the sample is decomposed into the disaccharide. In addition, the sample used for measuring the heparan sulfate is heated in the condition of the methanol decomposition reaction of the heparan sulfate described above. According to such a reaction, the heparan sulfate contained in the sample is decomposed into the disaccharide. In a case where the condition of the methanol decomposition reaction is the same, the sample that is a measurement target may be divided after the methanol decomposition reaction. In this case, only one of the dermatan sulfate and the heparan sulfate can be measured by being subjected to the methanol decomposition reaction.

The disaccharide obtained by decomposing the dermatan sulfate and/or the heparan sulfate with the methanol decomposition reaction (the methanolysis) is applied to liquid chromatography. Then, an eluate from the liquid chromatography is sequentially applied to mass analysis.

In this case, the liquid chromatography to be used is not particularly limited insofar as the disaccharide represented by Chemical Formulas (XII) and (XIV) described above can be separated from other substances by being once adsorbed in a column, and then, by being eluted.

For example, high-performance liquid column chromatography using a column filled with carriers capable of adsorbing the disaccharide by an ionic interaction, a hydrophobic interaction, a hydrophilic interaction, and the like can be preferably used in the method of the present invention.

The disaccharide that is exemplified by chemical reaction equations (XI) and (XIII) described above and is generated by the methanol decomposition reaction has high polarity. Accordingly, a carrier capable of retaining the disaccharide a hydrophilic interaction is particularly preferable as the carrier capable of once adsorbing the disaccharide, and then, of eluting the disaccharide. That is, high-performance liquid chromatography using the carrier capable of retaining the disaccharide by the hydrophilic interaction is preferable as a method for separating the disaccharide obtained

with the methanol decomposition reaction. Examples of the high-performance liquid chromatography include hydrophilic interaction liquid chromatography used in Example 4 as described below.

A flow path from an outlet of the liquid chromatography is connected to a mass spectrometer, and the eluate from the liquid chromatography is sequentially sent to the mass analysis.

In this case, the mass spectrometer to be used is not particularly limited. For example, in the mass spectrometer, any ionization method including a photoionization method (APPI), an electronic ionization method (EI), a chemical ionization method, an electron desorption method, a fast atom bombardment method (FAB), a matrix-assisted laser desorption/ionization method (MALDI), and an electrospray ionization method (ESI) may be adopted as an ion source for ionizing molecules that are an analysis target. In addition, in the mass spectrometer, an analysis unit for separating ionized molecules may be any type including a magnetic field deflection type, a quadrupole type, an ion trap type, and a tandem quadrupole type.

A mass spectrometer including an ion source operated in an electrospray ionization method (ESI) that is operated in a cation mode and a tandem quadrupole type analysis unit can be preferably used in the method of the present invention. A tandem quadrupole type mass spectrometer is a mass spectrometer in which a quadrupole (Q1) functioning as a mass filter, a quadrupole (Q2) functioning as a collision cell, and a quadrupole (Q3) functioning as a mass filter are arranged in series. In the quadrupole (Q1), target precursor ions are separated from various ions generated by ionization, on the basis of a mass charge ratio (m/z) of the ions. Next, in the collision cell (Q2), the precursor ions collide with inactive gas (for example, argon) or the like, and thus, product ions (fragment ions) are generated. Next, in the quadrupole (Q3), the obtained product ions are selectively detected on the basis of the mass charge ratio (m/z).

Hereinafter, it will be exemplified that a specific example of a method for generating the precursor ions and the product ions from the disaccharide obtained by the methanol decomposition of the dermatan sulfate represented by Formula (XI) described above, byn the tandem quadrupole type mass spectrometer. First, precursor ions having a mass charge ratio (m/z) of 426 can be obtained by ionizing the disaccharide. The precursor ions are represented by (XV) described below.

[Chem. 15]

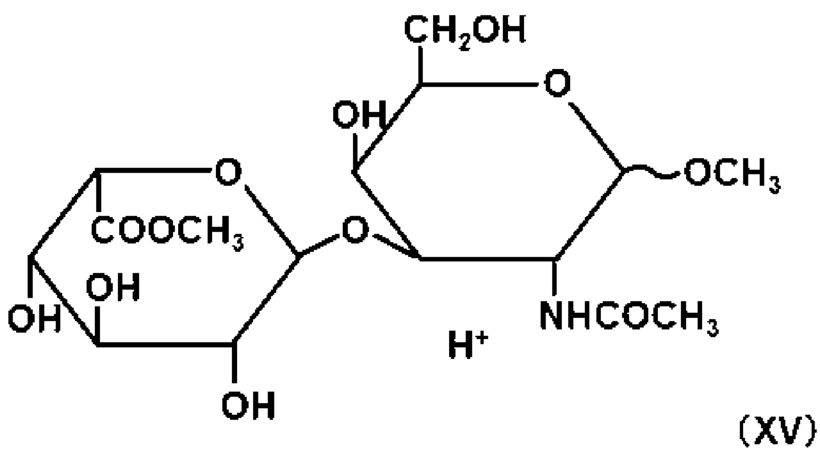

(XV)

The precursor ions represented by Formula (XV) described above are separated, and are cleaved in the collision cell (Q2), and thus, product ions having a mass charge ratio (m/z) of 236 can be obtained. A reaction for obtaining the product ions by cleaving the precursor ions is schematically represented by Formula (XVI).

[Chem. 16]

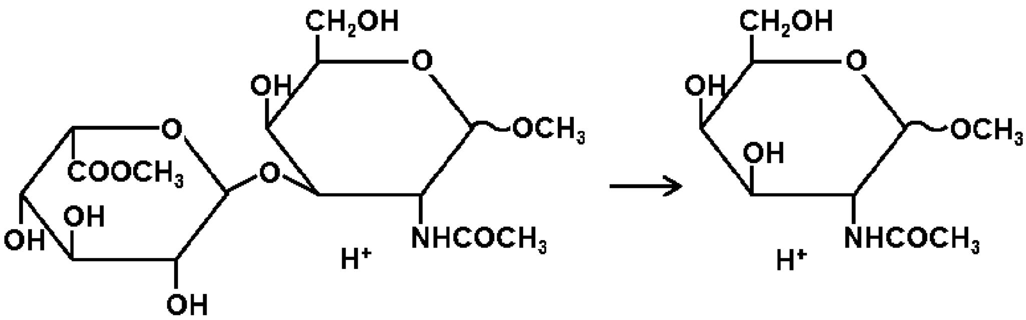

(XVI)

Hereinafter, it will be a exemplified that specific example of a method for generating the precursor ions and the product ions from the disaccharide by the methanol decomposition of the heparan sulfate represented by Formula (XIII) described above, by the tandem quadrupole type mass spectrometer. First, precursor ions having a mass charge ratio (m/z) of 384 can be obtained by ionizing the disaccharide. The precursor ions are represented by Formula (XVII) described below.

[Chem. 17]

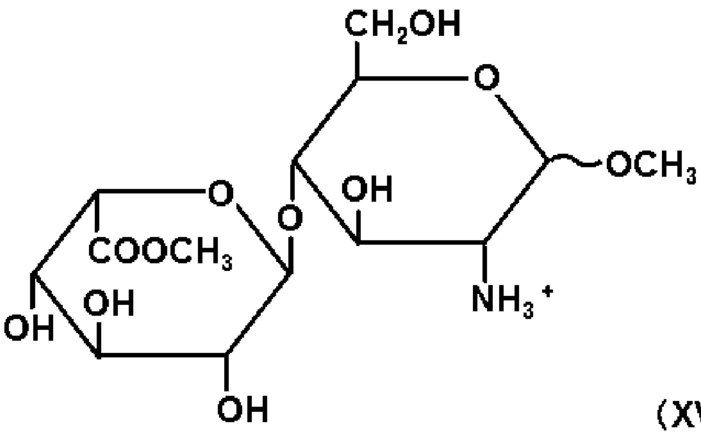

(XVII)

The precursor ions represented by Formula (XVII) described above are separated and are cleaved in the collision cell (Q2), and thus, product ions having a mass charge ratio (m/z) of 162 can be obtained. A reaction for obtaining the product ions by cleaving the precursor ions is schematically represented by Formula (XVIII).

[Chem. 18]

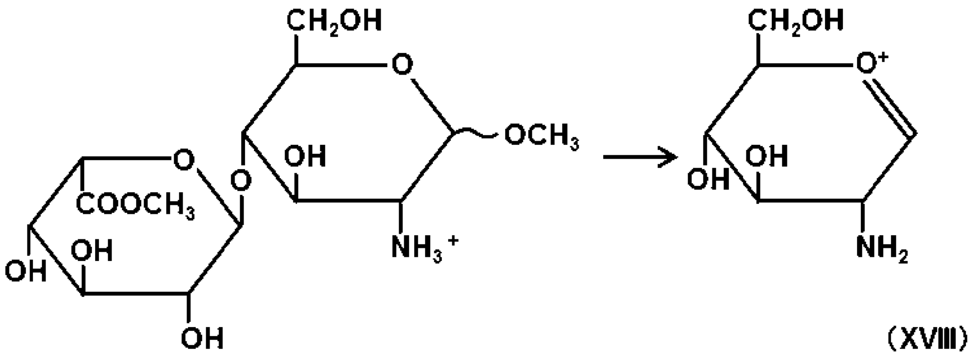

(XVIII)

The amount of dermatan sulfate contained in the sample can be measured by a method using the liquid chromatography and the tandem quadrupole type mass spectrometer described above. A known amount of dermatan

sulfate is subjected to the methanol decomposition and is analyzed by using the tandem quadrupole type mass spectrometer, and thus, the area of a peak (a detection peak) to be detected on a chromatography chart corresponding to product ions having a mass charge ratio (m/z) of 236 is calculated. Then, a standard curve representing a correlation between the amount of dermatan sulfate and the area of the detection peak is prepared. In addition, likewise, a sample in which the content of dermatan sulfate is unknown is subjected to the methanol decomposition and is analyzed by using the tandem quadrupole type mass spectrometer, and thus, the area of the detection peak corresponding to the product ions is calculated. The obtained value is interpolated into the standard curve, and thus, the dermatan sulfate contained in the sample can be measured.

Likewise, the amount of heparan sulfate contained in the sample can also be measured. A known amount of heparan sulfate is subjected to the methanol decomposition and is analyzed by using the tandem quadrupole type mass spectrometer, and thus, the area of a detection peak corresponding to product ions having a mass charge ratio (m/z) of 162 is calculated. Then, a standard curve representing a correlation between the amount of heparan sulfate and the area of the detection peak is prepared. In addition, likewise, a sample in which the content of the heparan sulfate is unknown is subjected to the methanol decomposition and is analyzed by using the tandem quadrupole type mass spectrometer, and thus, the area of the detection peak corresponding to the product ions is calculated. The obtained value is interpolated into the standard curve, and thus, the heparan sulfate contained in the sample can be measured.

The amount of chondroitin sulfate and heparan sulfate contained in the sample can be measured by combining the measurement methods for the chondroitin sulfate and the heparan sulfate described above. In this case, the sample that is a measurement target is divided, one of the divided samples is used as a sample for measuring the chondroitin sulfate, and the other one is used as a sample for measuring the heparan sulfate. The sample used for measuring the chondroitin sulfate is heated in the condition of the methanol decomposition reaction of the chondroitin sulfate described above. In addition, the sample used for measuring the heparan sulfate is heated in the condition of the methanol decomposition reaction of the heparan sulfate described above. In a case where the condition of the methanol decomposition reaction is the same, the sample that is a measurement target may be divided after the methanol decomposition reaction. Each methanol decomposition product of the chondroitin sulfate and the heparan sulfate is applied to the liquid chromatography to obtain the eluate, and the eluate is sequentially analyzed with the mass spectrometer, and thus, the amount of chondroitin sulfate and heparan sulfate contained in the sample can be measured.

The amount of chondroitin sulfate, dermatan sulfate, and the heparan sulfate contained in the sample can be measured by combining the measurement methods for chondroitin sulfate, dermatan sulfate, and the heparan sulfate described above. In this case, the sample that is a measurement target is divided, one of the divided samples is used as a sample for measuring the chondroitin sulfate, another is used as a sample for measuring the dermatan sulfate, and the other is used as a sample for measuring the heparan sulfate. The sample for measuring the chondroitin sulfate is heated in the condition of the methanol decomposition reaction of the chondroitin sulfate described above. The sample used for measuring dermatan sulfate is heated in the condition of the methanol decomposition reaction of the dermatan sulfate described above. In addition, the sample used for measuring the heparan sulfate is heated in the condition of the methanol decomposition reaction of the heparan sulfate described above. In a case where the condition of the methanol decomposition reaction is the same, the sample that is a measurement target may be divided after the methanol decomposition reaction. Each methanol decomposition product of the chondroitin sulfate, the dermatan sulfate, and the heparan sulfate is applied to the liquid chromatography to obtain the eluate, and the eluate is sequentially analyzed with the mass spectrometer, and thus, the amount of chondroitin sulfate, dermatan sulfate, and the amount of heparan sulfate contained in the sample can be measured.

In the present invention, the sample that is an analysis target is not particularly limited, and examples thereof include body fluid, a cell, a tissue, an organ, a cell culture solution, a tissue culture solution, food, and feed, or a derivative therefrom.

In a case where the sample is a cell, organism species from which the cell is derived is not particularly limited. The organism species may be a procaryote or an eucaryote, and for example, is a bacterium, a yeast, a plant, a bird, an amphibian, a reptile, and a mammal. In a case where the organism species is a mammal, the animal species are not particularly limited, and for example, are a human, a monkey, a mouse, a rat, a guinea pig, a hamster, a rabbit, a horse, a cow, a pig, a dog, and a cat, in particular, the animal species are a human.

In a case where the sample is body fluid, a tissue, or an organ, organism species from which the body fluid, the tissue, or the organ is derived are not particularly limited. Examples of the organism species include a plant, a bird, an amphibian, a reptile, and a mammal, and in particular, the organism species are a mammal. In a case where the organism species are a mammal, animal species are not particularly limited, and for example, are a human, a monkey, a mouse, a rat, a guinea pig, a hamster, a rabbit, a horse, a cow, a pig, a dog, and a cat, and in particular, the animal species are a human.

In a case where the sample is derived from a mammal, the sample, for example, is body fluid, blood, blood serum, blood plasma, bone marrow fluid, cerebral spinal fluid, and urine obtained from the mammal, or is derived therefrom. Here, the body fluid indicates general liquid components derived from a mammal, including blood, bone

marrow fluid, cerebral spinal fluid, saliva, tears, sweat, semen, synovial fluid, and urine. The blood serum or the blood plasma can be prepared from the blood by a known method such as centrifugal separation.

In addition, in a case where the sample is derived from a mammal, the sample, for example, is a cell, a tissue, and an organ obtained from the mammal, or is derived therefrom. Here, the type of cell is not particularly limited, and examples of the cell include a mesenchymal stem cell, a nerve cell, a neuroblast, a myoblast cell, a neuroglial cell, a Schwann cell, a cardiac muscle cell, a skeletal muscle cell, a smooth muscle cell, a cartilage cell, an osteoblast cell, a fibroblast cell, a keratinocyte, an epidermal cell, an endothelial cell, a corneal epidermal cell, a corneal endothelial cell, a retina cell, a liver cell, a mesangial cell, a mesenchymal cell, a blood cell, a blood-forming cell, a dendritic cell, and an interstitial cell. In addition, the tissue is not also particularly limited, and examples of the tissue include an epidermal tissue, a connective tissue, a muscle tissue, a nerve tissue, a fibrous connective tissue, a cartilage tissue, and a hypophysis bone tissue. In addition, the organ is not also particularly limited, and examples of the organ include a stomach, a small intestine, a large intestine, a liver, a pancreas, a kidney, a spleen, a heart, a lung, a pituitary, a testicle, an ovary, a cerebellum, a cerebrum, a interbrain, a midbrain, and a medulla oblongata.

Disorders in which any one or a plurality of chondroitin sulfate, dermatan sulfate, and heparan sulfate are accumulated in the body are known. Examples of the disorder include a Hunter syndrome, a Hurler syndrome, a Scheie syndrome, a Hurler-Scheie syndrome, Sanfilippo syndrome (types A to D), a Morquio syndrome (types A and B), a Maroteaux-Lamy syndrome, a Sly syndrome, and the like. In such a disorder, a method of treatment for alleviating a symptom by reducing any one or a plurality of such substances abnormally accumulated in the body has been tested. An enzyme replacement therapy for dosing the body with an enzyme for decomposing any one or a plurality of the chondroitin sulfate, the dermatan sulfate, and the heparan sulfate is an example of such a method of treatment.

For a patient with the disorder in which any one or a plurality of the chondroitin sulfate, the dermatan sulfate, and the heparan sulfate are accumulated in the body, the amount of any one or a plurality of substances accumulated in the body is measured, and screening can be performed on the basis of a measurement value that is obtained. In a case where the measurement value is abnormally higher than that of a normal human, it can be determined that the subject is the patient with the disorder. The method of the present invention can be implemented in order to perform such determination.

When the patient with the disorder in which any one or a plurality of the chondroitin sulfate, the dermatan sulfate, and the heparan sulfate are accumulated in the body is subjected to a medical treatment, the effect of the medical treatment can be checked by measuring the amount of substances accumulated in the body of the patient before and after the medical treatment, and by measuring a reduction amount after the medical treatment. It can be determined that the larger the reduction amount is, the higher the effect of the medical treatment. The method of the present invention can be implemented in order to check such an effect of the medical treatment. For example, it can be determined that the effect of the medical treatment is obtained in a case where the amount of one or a plurality of chondroitin sulfate, the dermatan sulfate, and the heparan sulfate accumulated in the body of the patient is reduced by preferably greater than or equal to 10%, more preferably greater than or equal to 20%, even more preferably greater than or equal to 30%, and still even more preferably greater than or equal to 50%, before and after the medical treatment, respectively.

In addition, the screening of a medical agent for the medical treatment of the patient with the disorder in which any one or a plurality of the chondroitin sulfate, the dermatan sulfate, and the heparan sulfate are accumulated in the body can be performed by dosing an experimental animal with an test drug, by measuring the amount of substances accumulated in the body of the experimental animal before and after the dose of test drug, and by measuring a reduction amount after the dose. It can be determined that the larger the reduction amount is, the higher the effect of the medical treatment of the test drug is. For example, it can be determined that the medical treatment of the test drug is effective in a case where the amount of substances accumulated in the body of the experimental animal after treating the test drug is reduced by preferably greater than or equal to 10%, more preferably greater than or equal to 20%, even more preferably greater than or equal to 30%, and still even more preferably greater than or equal to 50%, compared with the amount of substances measured before treating the test drug.

The Hunter syndrome is a disorder in which a part or all of iduronate-2-sulfatase activity is genetically lacked. The dermatan sulfate and the heparan sulfate are accumulated in the body of the patient by the defect or deletion of iduronate-2-sulfatase. Accordingly, the method of the present invention can be used in the screening of a patient with the Hunter syndrome, the check of the effect of a method of treatment thereof, the evaluation of a medicinal effect of a therapeutic agent, the screening of a test drug thereof, and the like. Examples of the therapeutic agent of the Hunter syndrome include human iduronate-2-sulfatase (hIDS), an analog of hIDS, and a bonded body of hIDS and an antibody, but the therapeutic agent is not limited thereto. Examples of the bonded body of hIDS and the antibody include fusion protein of hIDS and an anti-human transferrin receptor antibody.

In addition, the Hurler syndrome, the Scheie syndrome, and the Hurler-Scheie syndrome are disorders in which a part or all of L-iduronidase activity is genetically lacked. The dermatan sulfate and the heparan sulfate are accumulated in the body of the patient by the defect or deletion of the L-iduronidase. Accordingly, the method of the present invention can be used in the screening of a patient with such a disorder, the check of the effect of a method of treatment, the

evaluation of a medicinal effect of a therapeutic agent, the screening of a test drug, and the like. Examples of the therapeutic agent for treatment of such a disorder include L-iduronidase, an analog of the L-iduronidase, and a bonded body of the L-iduronidase and an antibody, but the therapeutic agent is not limited thereto. Examples of the bonded body of the L-iduronidase and the antibody include fusion of the L-iduronidase and an anti-human transferrin receptor antibody.

In addition, the Maroteaux-Lamy syndrome is a disorder in which a part or all of N-acetyl galactosamine-4-sulfatase (ASB) activity is genetically lacked. In particular, the dermatan sulfate and the chondroitin sulfate are accumulated in the body of the patient by the defect or deletion of ASB. Accordingly, the method of the present invention can be used in the screening of a patient with the Maroteaux-Lamy syndrome, the check of the effect of a method of treatment thereof, the evaluation of a medicinal effect of a therapeutic agent thereof, the screening of a test drug thereof, and the like. Examples of the therapeutic agent of the Maroteaux-Lamy syndrome include ASB, an analog of ASB, and a bonded body of ASB and an antibody, but the therapeutic agent is not limited thereto. Examples of the bonded body of ASB and the antibody include fusion protein of ASB and an anti-human transferrin receptor antibody.

In addition, the Sanfilippo syndrome is sorted into a type A, a type B, a type C, and a type D, each type is a disorder in which a part or all of heparan sulfate N-sulfatase activity, α-N-acetyl glucosaminidase activity, acetyl CoA:a-glucosaminide N-acetyl transferase activity, and N-acetyl glucosamine-6-sulfate sulfatase activity is genetically lacked, respectively. In particular, the heparan sulfate is accumulated in the body of the patient by the defect or deletion of such enzymes. Accordingly, the method of the present invention can be used in the screening of a patient with the Sanfilippo syndrome, the check of the effect of a method of treatment thereof, the evaluation of a medicinal effect of a therapeutic agent thereof, the screening of a test drug thereof, and the like. Examples of the therapeutic agent of the Sanfilippo syndrome include such enzymes, analogs of such enzymes, and a bonded body of any one of such enzymes and an antibody, but the therapeutic agent is not limited thereto. Examples of the bonded body of the enzyme and the antibody include fusion protein of such enzymes and an anti-human transferrin receptor antibody.

In addition, the Sly syndrome is a disorder in which a part or all of β-glucuronidase activity is lacked. The chondroitin sulfate, the dermatan sulfate, and the heparan sulfate are accumulated in the body of the patient by the defect or deletion of the β-glucuronidase. Accordingly, the method of the present invention can be used in the screening of a patient with the Sly syndrome, the check of the effect of a method of treatment thereof, the evaluation of a medicinal effect of a therapeutic agent thereof, the screening of an test drug thereof, and the like. Examples of the therapeutic agent of the Sly syndrome include β-glucuronidase, an analog thereof, and a bonded body of the β-glucuronidase and an antibody, but the therapeutic agent is not limited thereto. Examples of the bonded body of the β-glucuronidase and the antibody include fusion protein of the β-glucuronidase and an anti-human transferrin receptor antibody.

The method of the present invention can also be used as a method for measuring the amount of chondroitin sulfate and heparan sulfate contained in food and feed, or the amount of dermatan sulfate in addition thereto. For example, the amount of chondroitin sulfate, heparan sulfate, and the like contained in food or feed ingested by a human or an animal other than the human is measured in advance, and thus, such an ingestion amount can also be controlled. For example, when a patient with the disorder described above is in a state in which the ingestion amount of chondroitin sulfate, the heparan sulfate, and the like is to be limited, the ingestion amount of chondroitin sulfate and the like can be suitably controlled by ingesting food in which the amount of chondroitin sulfate and the like is measured in advance with the method of the present invention.

[EXAMPLES]

Hereinafter, the present invention will be described in more detail, with reference to examples, but the present invention is not limited to the examples.

[Example 1: Preparation of Various Solutions]

Examples 1 to 6 described below relate to an analytical method for chondroitin sulfate. Solutions of (a) to (k) used in a test were prepared in the following procedure.

(a) Ammonium Carbonate Solution of 10%: 10 g of ammonium carbonate was dissolved in 100 mL of water for injection to be an ammonium carbonate solution of 10% was obtained.

(b) Deuterium Labeling Solvent: 240 μL of acetyl chloride was dropped into 1.5 mL of methanol-$d_4$ (manufactured by Sigma-Aldrich) in an ice bath to be a deuterium labeling solvent.

(c) MeCN/Water: 2 mL of water for injection and 18 mL of acetonitrile were mixed to be MeCN/water.

(d) Mobile Phase A: 25 mL of an ammonium formate aqueous solution of 1 M was added to and mixed with 475 mL of water for injection to be a mobile phase A.

(e) Mobile Phase B: 70 mL of a mobile phase A was mixed with 930 mL of acetonitrile to be a mobile phase B.

(f) Chondroitin Sulfate Standard Stock Solution (CS Standard Stock Solution): Chondroitin sulfate A (manufactured by Sigma-Aldrich) was weighed in a microtube of 1.5 mL, and was dissolved with water for injection, and thus, a solution having a concentration of 5.0 mg/mL was prepared. 15 μL of the prepared solution was dispensed to a screw cap tube of 0.5 mL, and was frozen (lower than or equal to -15°C) and stocked until use. The solution was a CS standard stock solution.

(g) Solution for Preparing a Standard Curve: 990 μL of water for injection was measured off, and 10 μL of a CS standard stock solution was added thereto, and thus, a solution containing chondroitin sulfate at a concentration of 50 μg/mL was prepared. The solution was diluted with water for injection, and thus, a solution containing chondroitin sulfate at a concentration of 5000 ng/mL was prepared. The solution was diluted with water for injection in two stages, and thus, a solution containing chondroitin sulfate at a concentration of 25 to 5000 ng/mL was prepared. The solution was a solution for preparing a standard curve.

(h) Dermatan Sulfate Standard Stock Solution (DS Standard Stock Solution): Chondroitin sulfate B (manufactured by Sigma-Aldrich) was weighed to a microtube of 1.5 mL, and was dissolved with water for injection, and thus, a solution having a concentration of 5.0 mg/mL was prepared. 15 μL of the prepared solution was dispensed to a screw cap tube of 0.5 mL, and was frozen (lower than or equal to -15°C) and was stocked until use. The solution was a DS standard stock solution.

(i) Dermatan Sulfate Internal Standard Solution (DS Internal Standard Solution): 40 μL of a DS standard stock solution was measured off in a borosilicic acid screw-top test tube, and a solvent was vapored in a nitrogen stream. 400 μL of a deuterium labeling solvent was added to a dried product, was stirred, and then, was subjected to a deuteriomethanolysis reaction at 65°C for 75 minutes. After the reaction, a solvent was vapored in a nitrogen stream. 500 μL of MeCN/water was added to a dried product, and then, it was sonicated for 30 minutes. 20 μL of the solution was dispensed to a screw cap tube of 0.5 mL, and was frozen (lower than or equal to -15°C) and stocked. The solution was a dermatan sulfate internal standard solution (a DS internal standard solution).

(j) Internal Standard Solution: 4 μL of a DS internal standard solution was added to 4 mL of methanol, and was stirred. Such a solution was an internal standard solution. This solution was prepared at the time of use.

(k) Cell Extraction Solution: 2961 μL of a saline solution and 30 μL of a protease inhibitor were added to 9 μL of polyoxyethylene (10) octyl phenyl ether to be a cell extraction solution.

[Example 2: Preparation of Cell Sample Solution]

Cultured cells were washed with PBS, and then, a cell extraction solution was added, and the cells were recovered in a 1.5 mL tube. The 1.5 mL tube was left to stand on the ice for 90 minutes, and then, a supernatant was recovered by centrifugation (at 12000 rpm and 4°C for 10 minutes). Water for injection was added to the cell extract in order to adjust a protein concentration thereof to 100 μg/mL to be a cell sample solution.

[Example 3: Methanolysis Reaction]

20 μL of each solution for preparing a standard curve prepared in Example 1 described above was measured off and was individually dispensed to each borosilicic acid screw-top test tube. In addition, water for injection as a blank was dispensed to the borosilicic acid screw-top test tube. A solvent in the test tube was vapored in a nitrogen stream (N = 1). 20 μL of 2,2-dimethoxypropane and 200 μL of HCl-methanol in which the concentration of HCl-methanol was 3 N were added to a dried product, were stirred, and then, were subjected to a methanolysis reaction by using a constant temperature water bath at 70°C for 90 minutes. After the reaction, the test tube was left to stand in ice, and then, 200 μL of an ammonium carbonate solution of 10% was added to stop the reaction. 50 μL of an internal standard solution was added, and then, a solvent was vapored in a nitrogen stream. 250 μL of water for injection was added to a dried product, was dissolved, and then, was purified by using a solid phase cartridge (OASIS HLB (1 cc, 30 mg)). A solvent was vapored in a nitrogen stream. 200 μL of MeCN/water was added to dissolve it, and thus, a supernatant was collected in a vial.

In addition, 20 μL of the cell sample solution prepared in Example 2 was measured off and was dispensed to a borosilicic acid screw-top test tube, and a solvent in the test tube was vapored in a nitrogen stream. 20 μL of 2,2-dimethoxypropane and 200 μL of HCl-methanol in which the concentration of HCl-methanol was 3 N were added to a dried product, were stirred, and then, were subjected to a methanolysis reaction by using a constant temperature water bath at 70°C for 90 minutes. After the reaction, the test tube was left to stand in ice, and then, 200 μL of an ammonium carbonate solution of 10% was added to stop the reaction. 50 μL of an internal standard solution was added, and then, a solvent was vapored in a nitrogen stream. 250 μL of water for injection was added to a dried product, was dissolved, and then, was purified by using a solid phase cartridge (OASIS HLB (1 cc, 30 mg)). A solvent was vapored in a nitrogen stream. 200 μL of MeCN/water was added to dissolve it, and thus, a supernatant was collected in a vial.

[Example 4: LC/MS/MS Analysis]

LC/MS/MS analysis was implemented by using a combination of hydrophilic interaction ultra-performance liquid chromatography and a tandem quadrupole type mass spectrometer. QTRAP5500 (manufactured by AB Sciex Pte. Ltd.) was used as the mass spectrometer (an MS/MS device), and Nexera X2 (manufactured by SHIMADZU CORPORATION) was set as an HPLC device. In addition, Acquity UPLC™ BEH Amide of 1.7 μm (2.1 × 150 mm, manufactured by Waters Corporation) was used as an LC column. The mobile phase A and the mobile phase B prepared in Example 1 were used as a mobile phase. In addition, a column temperature was set to 60°C.

The column was equilibrated with a mixed liquid containing the mobile phase A of 6% (v/v) and the mobile phase B of 94% (v/v), and then, 5 μL of a sample was injected, and chromatography was implemented in a gradient condition of the mobile phase shown in Table 1. Note that, a flow rate of the mobile phase was 0.4 mL/minute.

[Table 1]

| **TABLE 1. Conditions for liquid chromatography** | | |
|---|---|---|
| **Elapsed time after sample injection (min)** | **Mobile phase A (%(v/v))** | **Mobile phase B (%(v/v))** |
| **0** | **0** | **100** |
| **5** | **0** | **100** |
| **7** | **20** | **80** |
| **12.5** | **20** | **80** |
| **13** | **70** | **30** |
| **15** | **70** | **30** |
| **15.5** | **0** | **100** |
| **20** | **0** | **100** |

Ion source parameters of the MS/MS device were set as shown in Table 2, in accordance with an instruction leaflet of QTRAP5500 (manufactured by AB Sciex Pte. Ltd.).

[Table 2]

| **TABLE 2. Setting parameters of the ion source of the MS / MS Device** | |
|---|---|
| **Ion Source** | **ESI (TurboV)** |
| **Polarity** | **Positive** |
| **Scan type** | **MRM** |
| **IonSpray voltage** | **5500 V** |
| **Heater gas temperature** | **425° C** |
| **Curtain gas (CUR)** | **30.0 psi** |
| **Collision gas (CAD)** | **8 psi** |
| **Ion Source Gas 1 (GS1)** | **70.0 psi** |
| **Ion Source Gas 2 (GS2)** | **70.0 psi** |
| **Entrance Potential** | **10.0 V** |
| **Duration** | **14.994 min** |

The area of a peak (a detection peak) to be found on a chromatography chart of product ions derived from chondroitin sulfate contained in each of the solutions for preparing a standard curve was obtained by measuring the solution for preparing a standard curve. In addition, the area of a detection peak of product ions derived from a DS internal standard solution was obtained. The solution for preparing a standard curve was measured at N = 1.

Here, the product ions to be detected are ions obtained by Reaction equation (VI) described above. Note that, the dermatan sulfate contained in the DS internal standard solution is deuterium-labeled. Accordingly, a mass charge

ratio (m/z) of precursor ions derived therefrom is 432, and thus, is larger than that of unlabeled chondroitin sulfate. A mass charge ratio (m/z) of precursor ions derived from unlabeled chondroitin sulfate is 426. Accordingly, such precursor ions are separated in a quadrupole (Q1), on the basis of the mass charge ratio (m/z) of the ions, and thus, can be individually detected. (m/z) of the precursor ions and the product ions are collectively shown in Table 3.

An ion chromatogram obtained by LC/MS/MS analysis of a solution for preparing a standard curve containing chondroitin sulfate at a concentration of 250 ng/mL that is subjected to methanolysis is shown in Fig. 1. In Fig. 1, the amount of ions contained in an eluate from liquid chromatography is sequentially detected at m/z = 426 corresponding to precursor ions of the chondroitin sulfate. In the figure, a black-painted peak corresponds to the precursor ions of the chondroitin sulfate.

[Table 3]

| TABLE 3. (m/z) values of precursor ions and product ions | | |
|---|---|---|
| | **Precursor ion (m/z)** | **Product ion (m/z)** |
| **Chondroitin sulfate** | **426** | **236** |
| **Dermatan sulfate (deuterium-labeled)** | **432** | **239** |

An area ratio (CS Detection Peak Area/DS-IS Detection Peak Area) of a detection peak derived from the DS internal standard solution (a DS-IS detection peak area) to the area of the detection peak derived from the chondroitin sulfate (a CS detection peak area) contained in each of the solutions for preparing a standard curve was obtained. The value was taken on a vertical axis, the concentration of the chondroitin sulfate in each of the solutions for preparing a standard curve was taken on a horizontal axis, and a regression formula was calculated by using quadratic programming, and thus, a standard curve was prepared.

[Example 5: Study of Standard Curve]

The standard curve obtained from a measurement value of the solution for preparing a standard curve exhibited excellent linearity in a concentration range of 25.0 to 5000 ng/mL (Fig. 2). A correlation coefficient (r) was 0.9998.

[Example 6: Measurement Result of Chondroitin Sulfate Contained in Cultured Cell Extraction Liquid]

A cell sample solution that is an extract of the cultured cell prepared in Example 2 is subjected to a methanolysis reaction with the method described in Example 3, and then, is analyzed with the method described in Example 4, and thus, the area of the detection peak derived from the chondroitin sulfate (a cell sample solution detection peak area) can be obtained. Further, a ratio of the area to the DS-IS detection peak area (Cell Sample Solution Detection Peak Area/DS-IS Detection Peak Area) is obtained, and the ratio is interpolated to the standard curve obtained in Example 4, and thus, the chondroitin sulfate contained in the cell sample solution can be quantified.

From the results described above, a heating temperature of 70°C and a heating time of 90 minutes are preferable as a methanolysis condition of the chondroitin sulfate contained in the extract of the culture cell.

[Example 7: Preparation of Various Solutions]

Examples 7 to 14 described below relate to an analytical method for heparan sulfate and/or dermatan sulfate. Solutions of (1) to (w) used in a test were prepared in the following order.

(1) MeCN/Water: 0.5 mL of water for injection and 4.5 mL of acetonitrile were mixed to be MeCN/water. This solution was prepared at the time of use.
(m) Deuterium Labeling Solvent: 240 μL of acetyl chloride was dropped into 1.5 mL of methanol-$d_4$ (manufactured by Sigma-Aldrich) in an ice bath to be a deuterium labeling solvent. This solution was prepared at the time of use.
(n) PBS/Citric Acid Solution: By dissolving a citric acid in pure water, a citric acid solution having a concentration of 10 mM was prepared. Further, by dissolving a trisodium citrate dihydrate in pure water, a sodium citrate solution having a concentration of 10 mM was prepared. The sodium citrate solution was dropped into the citric acid solution such that pH was adjusted to 3.0. The solution was a citric acid buffer solution of 10 mM (pH 3.0). In addition, a solution in which 2 mL of PBS was added to 18 mL of the citric acid buffer solution (pH 3.0) of 10 mM was a PBS/citric acid solution.
(o) Mobile Phase A: 2.5 mL of an ammonium formate aqueous solution of 1 M and 400 μL of an ammonium hydroxide

aqueous solution ($NH_4OH$ of 25%) were added to and mixed with 247.5 mL of pure water to be a mobile phase A. This solution was prepared at the time of use.

(p) Mobile Phase B: 5 mL of an ammonium formate aqueous solution of 1 M, 450 mL of acetonitrile, and 800 μL of an ammonium hydroxide solution ($NH_4OH$ of 25%) were mixed with 45 mL of pure water to be a mobile phase B. This solution was prepared at the time of use.

(q) Heparan Sulfate Standard Stock Solution (HS standard Stock Solution): Heparan sulfate (manufactured by Iduron Inc.) was weighed in a microtube of 1.5 mL, and was dissolved with water for injection, and thus, a solution having a concentration of 5.0 mg/mL was prepared. 15 μL of the prepared solution was dispensed to a screw cap tube of 0.5 mL, and was frozen (lower than or equal to -15°C) and stocked until use. The solution was an HS standard stock solution.

(r) Dermatan Sulfate Standard Stock Solution (DS Standard Stock Solution): Chondroitin sulfate B sodium salt from porcine intestinal mucosa (manufactured by Sigma-Aldrich) was weighed in a microtube of 1.5 mL, and was dissolved with water for injection, and thus, a solution having a concentration of 5.0 mg/mL was prepared. 15 μL of the prepared solution was dispensed to a screw cap tube of 0.5 mL, and was frozen (lower than or equal to -15°C) and stocked until use. The solution was a DS standard stock solution.

(s) Dermatan Sulfate Internal Standard Solution (DS Internal Standard Solution): 40 μL of a DS standard stock solution was measured off in a borosilicic acid screw-top test tube, and a solvent was vapored in a nitrogen stream. 400 μL of a deuterium labeling solvent was added to a dried product, was stirred, and then, was subjected to a deuteriomethanolysis reaction at 65°C for 75 minutes. After the reaction, a solvent was vapored in a nitrogen stream. 500 μL of MeCN/water was added to a dried product, and it was sonicated for 30 minutes. 20 μL of a solution that was prepared was dispensed to a screw cap tube of 0.5 mL, and was frozen (lower than or equal to - 15°C) and stocked. The solution was a dermatan sulfate internal standard solution (a DS internal standard solution).

(t) Heparan Sulfate Internal Standard Solution (HS Internal Standard Solution) : 40 μL of an HS standard stock solution was measured off in a borosilicic acid screw-top test tube, and a solvent was vapored in a nitrogen stream. 400 μL of a deuterium labeling solvent was added to a dried product, was stirred, and then, was subjected to a deuteriomethanolysis reaction at 65°C for 75 minutes. After the reaction, a solvent was vapored in a nitrogen stream. 500 μL of MeCN/water was added to a dried product, and it was sonicated for 30 minutes. 20 μL of the solution was dispensed to a screw cap tube of 0.5 mL, and was frozen (lower than or equal to -15°C) and stocked. The solution was a heparan sulfate internal standard solution (an HS internal standard solution).

(u) Solution for Dissolving Sample: 1 μL of an HS internal standard solution and 1 μL of a DS internal standard solution were added to 5 mL of MeCN/water, were stirred, and then, were subjected to an ultrasonic treatment for 30 minutes. The solution was a solution for dissolving a sample. This solution was prepared at the time of use.

(v) Solution for Preparing Standard Curve: 480 μL of a PBS/citric acid solution was measured off, and 10 μL of an HS standard stock solution and 10 μL of a DS standard stock solution were added thereto, and thus, a solution containing 100 μg/mL of dermatan sulfate and 100 μg/mL of heparan sulfate was prepared. The solution was diluted with a PBS/citric acid solution, and thus, a solution containing 2500 ng/mL of dermatan sulfate and 2500 ng/mL of heparan sulfate was prepared. The solution was diluted with a PBS/citric acid solution in two stages, and thus, a solution containing dermatan sulfate and heparan sulfate at a concentration of 25 to 2500 ng/mL, respectively, was prepared. The solution was a solution for preparing a standard curve.

(w) Tissue Extraction Solution A saline solution was added to 1 mL of polyoxyethylene (10) octyl phenyl ether such that the entire amount was 500 mL. The solution was a tissue extraction solution.

[Example 8: Preparation of Blood Sample]

Blood was collected from a wild type mouse (C57BL/6N) and a hemizygote mouse lacking a chromosomal region having an IDS gene (an IDS hemizygote mouse, C57BL/6N). The collected blood was moved to a tube, and was left to stand at a room temperature for longer than or equal to 30 minutes, and then, blood serum was recovered by centrifugation (2000 g, for 20 minutes). 8 μL of PBS and 144 μL of a citric acid buffer solution (pH 3.0) of 10 mM were added to 8 μL of the blood serum, and were stirred. 20 μL thereof was measured off and was dispensed to a borosilicic acid screw-top test tube. This was a blood sample solution.

[Example 9: Methanolysis Reaction]

20 μL of each solution for preparing a standard curve prepared in Example 7 described above was measured off and was individually dispensed to each borosilicic acid screw-top test tube. In addition, a PBS/citric acid solution as a blank was dispensed to the borosilicic acid screw-top test tube. A solvent in the test tube was vapored in a nitrogen stream (N = 1). 20 μL of 2,2-dimethoxypropane and 200 μL of HCl-methanol in which the concentration of HCl-methanol was 3 N were added to a dried product, were stirred, and then, were subjected to a methanolysis reaction in three

conditions (conditions A to C) shown in Table 4. After the reaction, a solvent was vapored in a nitrogen stream. 50 μL of the solution for dissolving a sample was added to a dried product to dissolve it, and then, was subjected to centrifugation (at 15000 rpm and a room temperature for 10 minutes), and thus, a supernatant was collected in a vial.

In addition, a dermatan sulfate standard stock solution and a heparan sulfate standard stock solution were diluted with a PBS/citric acid solution, and four types of quality control samples (QC samples) containing a dermatan sulfate standard stock solution and a heparan sulfate standard stock solution at a concentration of 25.0 ng/mL, 50.0 ng/mL, 500 ng/mL, and 2000 ng/mL, respectively, were prepared to be QC-LL, QC-L, QC-M, and QC-H, respectively. 20 μL of each of the samples was measured off and was individually dispensed to a borosilicic acid screw-top test tube. A solvent in the test tube was vapored in a nitrogen stream (N = 1). 20 μL of 2,2-dimethoxypropane and 200 μL of HCl-methanol in which the concentration of HCl-methanol was 3 N were added to a dried product, were stirred, and then, were subjected to a methanolysis reaction in three types of conditions (conditions A to C) shown in Table 4. After the reaction, a solvent was vapored in a nitrogen stream. 50 μL of the solution for dissolving a sample was added to a dried product to dissolve it, and then, was subjected to centrifugation (at 15000 rpm and a room temperature for 10 minutes), and thus, a supernatant was collected in a vial.

In addition, 20 μL of the blood sample solution prepared in Example 8 was measured off and was dispensed to a borosilicic acid screw-top test tube. A solvent in the test tube was vapored in a nitrogen stream (N = 1). 20 μL of 2,2-dimethoxypropane and 200 μL of HCl-methanol in which the concentration of HCl-methanol was 3 N were added to a dried product, were stirred, and then, were subjected to a methanolysis reaction in three types of conditions (conditions A to C) shown in Table 4. After the reaction, a solvent was vapored in a nitrogen stream. 50 μL of the solution for dissolving a sample was added to a dried product to dissolve it, and then, was subjected to centrifugation (at 15000 rpm and a room temperature for 10 minutes), and thus, a supernatant was collected in a vial.

[Table 4]

| TABLE 4. Conditions for methanolysis reaction | | |
|---|---|---|
| | **Heating Temperature (°C)** | **Heating Time** |
| **Condition A** | **65** | **75 minutes** |
| **Condition B** | **80** | **2 hours** |
| **Condition C** | **65** | **18 hours** |

[Example 10: LC/MS/MS Analysis]

LC/MS/MS analysis was implemented by using a combination of hydrophilic interaction ultra-performance liquid chromatography and a tandem quadrupole type mass spectrometer. QTRAP5500 (manufactured by AB Sciex Pte. Ltd.) was used as the mass spectrometer (an MS/MS device), and Nexera X2 (manufactured by SHIMADZU CORPORATION) was set as an HPLC device. In addition, Acquity UPLC™ BEH Amid of 1.7 μm (2.1 × 50 mm, manufactured by Waters Corporation) was used as an LC column. The mobile phase A and the mobile phase B prepared in Example 7 were used as a mobile phase. In addition, a column temperature was set to 50°C.

The column was equilibrated with a mixed liquid containing the mobile phase A of 6% (v/v) and the mobile phase B of 94% (v/v), and then, 10 μL of a sample was injected, and chromatography was implemented in a gradient condition of the mobile phase shown in Table 5. Note that, a flow rate of the mobile phase was 0.4 mL/minute.

[Table 5]

| TABLE 5. Conditions for liquid chromatography | | |
|---|---|---|
| **Elapsed time after sample injection (min)** | **Mobile phase A (%(v/v))** | **Mobile phase B (%(v/v))** |
| **0** | **6** | **94** |
| **1** | **6** | **94** |
| **4** | **30** | **70** |
| **4.01** | **6** | **94** |
| **6** | **6** | **94** |

Ion source parameters of the MS/MS device were set as shown in Table 6, in accordance with an instruction

leaflet of QTRAP5500 (manufactured by AB Sciex Pte. Ltd.).

[Table 6]

| TABLE 6. Setting parameters of the ion source of the MS / MS instrument | |
|---|---|
| Ion Source | ESI (TurboV) |
| Polarity | Positive |
| Scan type | MRM |
| IonSpray voltage | 5500 V |
| Heater gas temperature | 500°C |
| Curtain gas (CUR) | 30.0 psi |
| Collision gas (CAD) | 8 psi |
| Ion Source Gas 1 (GS1) | 70.0 psi |
| Ion Source Gas 1 (GS2) | 70.0 psi |
| Entrance Potential | 10.0 V |
| Duration | 4.00 min |

The area of a peak (a detection peak) to be found on a chromatography chart of product ions derived from dermatan sulfate and heparan sulfate contained in each of the solutions for preparing a standard curve was obtained by measuring the solution for preparing a standard curve and the QC sample. In addition, the area of a detection peak of product ions derived from a DS internal standard solution and an HS internal standard solution. The solution for preparing a standard curve was measured at N = 1, and the QC samples (QC-LL, QC-L, QC-M, and QC-H) were measured at N = 3.

Here, the product ions to be detected are ions obtained by Reaction equation (XVI) of the dermatan sulfate described above, and are ions obtained by Reaction equation (XVIII) of the heparan sulfate described above. Note that, the dermatan sulfate and the heparan sulfate contained in the DS internal standard solution and the HS internal standard solution are deuterium-labeled. Accordingly, mass charge ratios (m/z) of precursor ions derived therefrom are 432 and 390, respectively, and thus, are larger than that of unlabeled dermatan sulfate and heparan sulfate. Mass charge ratios (m/z) of precursor ions derived from the unlabeled dermatan sulfate and heparan sulfate are 426 and 384, respectively. Accordingly, such precursor ions are separated in a quadrupole (Q1), on the basis of the mass charge ratio (m/z) of the ions, and thus, can be individually detected. (m/z) of the precursor ions and the product ions are collectively shown in Table 7.

An ion chromatogram obtained by LC/MS/MS analysis of a solution for preparing a standard curve containing dermatan sulfate and heparan sulfate at a concentration of 25 ng/mL that is subjected to methanolysis in a condition A shown in Table 4 is shown in Fig. 3. In Fig. 3, the amount of ions contained in an eluate from liquid chromatography is sequentially detected at m/z = 426 corresponding to precursor ions of the dermatan sulfate. In the drawing, a black-painted peak corresponds to the precursor ions of the dermatan sulfate.

In addition, an ion chromatogram obtained by LC/MS/MS analysis of a solution for preparing a standard curve containing dermatan sulfate and heparan sulfate at a concentration of 25 ng/mL that is subjected to methanolysis in a condition B shown in Table 4 is shown in Fig. 4. In Fig. 4, the amount of ions contained in an eluate from liquid chromatography is sequentially detected at m/z = 384 corresponding to precursor ions of the heparan sulfate. In the drawing, a black-painted peak corresponds to the precursor ions of the heparan sulfate.

[Table 7]

| TABLE 7. (m/z) values of precursor ion and product ion | | |
|---|---|---|
| | Precursor ion (m/z) | Product ion (m/z) |
| Dermatan sulfate | 426 | 236 |
| Dermatan sulfate (deuterium-labeled) | 432 | 239 |
| Heparan sulfate | 384 | 162 |
| Heparan sulfate (deuterium-labeled) | 390 | 162 |

An area ratio of a detection peak derived from the DS internal standard solution (a DS-IS detection peak area) to the area of the detection peak derived from the dermatan sulfate (a DS detection peak area) contained in each of the solutions for preparing a standard curve (DS Detection Peak Area/DS-IS Detection Peak Area) was obtained. The value was taken on a vertical axis, the concentration of the dermatan sulfate in each of the solutions for preparing a standard curve was taken on a horizontal axis, and a regression formula was calculated by using quadratic programming, and thus, a standard curve was prepared.

In addition, an area ratio of a detection peak derived from the HS internal standard solution (an HS-IS detection peak area) to the area of the detection peak derived from the heparan sulfate (an HS detection peak area) contained in each of the solutions for preparing a standard curve (HS Detection Peak Area/HS-IS Detection Peak Area) was obtained. The value was taken on a vertical axis, the concentration of the heparan sulfate in each of the solutions for preparing a standard curve was taken on a horizontal axis, and a regression formula was calculated by using quadratic programming, and thus, a standard curve was prepared.

In addition, an accuracy (%) and a trueness (%) were obtained by the following calculation formula, on the basis of the measurement values of the QC samples.

Accuracy (%) = Standard Deviation of Measurement Value/Average Value of Measurement Value × 100

Trueness (%) = Average Value of Measurement Value/Theoretical Concentration × 100

Note that, the theoretical concentration in the calculating formula of the trueness (%) means the concentration of the dermatan sulfate or the heparan sulfate added to the QC samples.

[Example 11: Consideration of Standard Curve Obtained in Condition A]

The standard curve (the standard curve of the condition A) obtained from a measurement value of the solution for preparing a standard curve subjected to methanolysis in the condition A shown in Table 4 described above exhibited excellent linearity in a concentration range of 25.0 to 2500 ng/mL (data is not shown), in both of the dermatan sulfate and the heparan sulfate. A correlation coefficient (r) was 0.9995 in the dermatan sulfate, and was 0.9938 in the heparan sulfate.

Next, the accuracy and the trueness will be considered. In Table 8 and Table 9, the accuracy and the trueness in each of the QC samples are shown. These values indicate that the condition A is preferable as a methanolysis condition for measuring the concentration of the dermatan sulfate and the heparan sulfate in the concentration range of 25.0 to 2500 ng/mL. Here, the measurement value of the heparan sulfate at a concentration of 25.0 ng/mL was considerably reduced in both of the trueness and the accuracy (Table 8). On the other hand, in the dermatan sulfate, the trueness and the accuracy of the measurement value at a concentration of 25.0 ng/mL were not considerably reduced (Table 9).

[Table 8]

**TABLE 8. Precision and trueness of measurements of heparan sulfate in QC samples**

| QC sample | Concentration of heparan sulfate (ng/mL) | Precision (%) | Trueness (%) |
|---|---|---|---|
| QC-LL | 25.0 | 27.8 | 65.9 |
| QC-L | 50.0 | 10.2 | 57.4 |
| QC-M | 500 | 9.7 | 102.4 |
| QC-H | 2000 | 6.1 | 95.1 |

[Table 9]

**TABLE 9. Precision and trueness of measurements of dermatan sulfate in QC samples**

| QC sample | Concentration of dermatan sulfate (ng/mL) | Precision (%) | Trueness (%) |
|---|---|---|---|
| QC-LL | 25.0 | 13.6 | 104.1 |

(continued)

| TABLE 9. Precision and trueness of measurements of dermatan sulfate in QC samples | | | |
|---|---|---|---|
| **QC sample** | **Concentration of dermatan sulfate (ng/mL)** | **Precision (%)** | **Trueness (%)** |
| **QC-L** | **50.0** | **6.2** | **88.4** |
| **QC-M** | **500** | **4.6** | **98.5** |
| **QC-H** | **2000** | **3.8** | **98.4** |

[Example 12: Comparison in Standard Curves Obtained from Conditions A, B, and C]

All of the standard curves obtained from the measurement values of the solutions for preparing a standard curve subjected to methanolysis in conditions A, B, and C exhibited excellent linearity in a concentration range of 25 to 2500 ng/mL (data is not shown), in both of the dermatan sulfate and the heparan sulfate. Correlation coefficients (r) in the standard curves in each of the conditions are shown in Table 10.

[Table 10]

| TABLE 10. Correlation coefficient (r) of calibration curve under conditions A, B and C | | |
|---|---|---|
| | **Correlation coefficient (r)** | |
| | **Dermatan sulfate** | **Heparan sulfate** |
| **Condition A** | **0.9995** | **0.9938** |
| **Condition B** | **0.9970** | **0.9993** |
| **Condition C** | **0.9972** | **0.9999** |

However, in Example 11, in the condition A, both of the trueness and the accuracy of the measurement value of the heparan sulfate at a concentration of 25.0 ng/mL were reduced. Accordingly, a condition for more accurately measuring the heparan sulfate at a concentration of 25.0 ng/mL was considered. Therefore, the trueness and the accuracy of the measurement value of the heparan sulfate of the QC sample (QC-LL) were compared in the conditions A, B, and C. Note that, the QC sample (QC-LL) contains both of the dermatan sulfate and the heparan sulfate at a concentration of 25.0 ng/mL. Results thereof are shown in Table 11. In the heparan sulfate, a high measurement value was obtained in both of the trueness and the accuracy in the case of being measured in the condition B. In the heparan sulfate, a high measurement value was obtained in both of the trueness and the accuracy even in the case of being measured in the condition C, but in the condition C, a thermal treatment takes a long time. Accordingly, it was concluded that the condition B is preferable as methanol decomposition for measuring the heparan sulfate. Note that, as described in Example 11, both of the trueness and the accuracy of the measurement value of the heparan sulfate were reduced in the condition A.

The trueness and the accuracy of the measurement value of the dermatan sulfate were also compared in the conditions A, B, and C. Results thereof are shown in Table 11. In the dermatan sulfate, both of the trueness and the accuracy were reduced in the conditions B and C, compared to the condition A.

Such results indicate that the condition A is preferable as methanol decomposition for measuring the dermatan sulfate, and the condition B is preferable as methanol decomposition for measuring the heparan sulfate.

[Table 11]

| TABLE 11. Precision and trueness of measurements of dermatan sulfate and heparan sulfate in QC sample (QC-LL) under conditions A, B and C | | | | |
|---|---|---|---|---|
| | **Dermatan sulfate** | | **Heparan sulfate** | |
| | **Precision (%)** | **Trueness (%)** | **Precision (%)** | **Trueness (%)** |
| **Condition A** | 1.0 | 101.2 | 9.1 | 125.1 |
| **Condition B** | 43.3 | 61.3 | 1.5 | 90.8 |
| **Condition C** | 24.1 | 121.9 | 0.2 | 105.7 |

[Example 13: Comparison between Measurement Results of Dermatan Sulfate and Heparan Sulfate Contained in Blood Serum in Conditions A, B, and C]

It was examined which of methanolysis condition A, B and C was preferable as a measurement method for dermatan sulfate and heparan sulfate contained in a sample derived from a living body by using a methanolysis reactant of the blood sample solution prepared from the blood of the wild type mouse and the IDS hemizygote mouse described in Example 9. Each methanolysis reactant was analyzed with the method described in Example 10, and the area of the detection peak derived from the dermatan sulfate (a blood sample solution DS detection peak area) was obtained. Further, a ratio of the area to the DS-IS detection peak area (Blood Sample Solution DS Detection Peak Area/DS-IS Detection Peak Area) was obtained, and the ratio was interpolated to the standard curve in the corresponding methanolysis reaction condition obtained in Example 10, and thus, the dermatan sulfate contained in the blood sample solution was quantified. In addition, each of the methanolysis reactants was analyzed with the method described in Example 10, and the area of the detection peak derived from the heparan sulfate (a blood sample solution HS detection peak area) was obtained, and thus, a ratio of the area to the HS-IS detection peak area (Blood Sample Solution HS Detection Peak Area/HS-IS Detection Peak Area) was obtained. The ratio was interpolated to the standard curve of the corresponding methanolysis reaction condition obtained in Example 10, and thus, the heparan sulfate contained in the blood sample solution was quantified. IDS is an enzyme having activity for decomposing GAG containing dermatan sulfate and heparan sulfate. Accordingly, it is known that the concentration of GAG in the blood of the IDS hemizygote mouse is higher than that of the wild type mouse.

A measurement result of the dermatan sulfate in the blood serum collected from the wild type mouse and the IDS hemizygote mouse is shown in Fig. 5, and a measurement result of the heparan sulfate is shown in Fig. 6, in the conditions A, B, and C.

In the condition A, the measurement value of the dermatan sulfate was higher in the IDS hemizygote mouse than in the wild type mouse (Fig. 5). Such a result reflects the genotype of the IDS hemizygote mouse. On the other hand, in the conditions B and C, the measurement value of the dermatan sulfate is approximately the same in the wild type mouse and the IDS hemizygote mouse, which was extremely higher than the measurement result of the dermatan sulfate in the condition A (Fig. 5). It is considered that such results are obtained because in the conditions B and C, product ions derived from components other than the dermatan sulfate contained in the blood serum are generated. That is, in the conditions B and C, it is difficult to measure the dermatan sulfate in the blood serum.

On the other hand, in any one of the conditions A to C, the measurement value of the heparan sulfate was higher in the IDS hemizygote mouse than in the wild type mouse, which was a measurement result reflecting the genotype of the IDS hemizygote mouse (Fig. 6). However, from the results shown in Table 11, it can be said that in the measurement value of the heparan sulfate, the accuracy of a value measured in the condition B is higher than that of a value measured in the condition A. Accordingly, it can be said that even in the measurement results in the conditions A to C shown in Fig. 6, both of the trueness and the accuracy of the measurement value in the condition B are higher than those of the measurement value in the condition A.

From the results described above, it can be concluded that the condition A (Heating Temperature: 65°C, Heating Time: 75 minutes) is preferable as a methanolysis condition of the dermatan sulfate contained in the blood serum, and the condition B (Heating Temperature: 80°C, Heating Time: 2 hours) is preferable as a methanolysis condition of the heparan sulfate.

[Example 14: Measurement of Dermatan Sulfate and Heparan Sulfate Contained in Mouse Blood Dosed with IDS]

Recombinant human iduronate-2-sulfatase (rhIDS) commercially available for medical use was diluted with a saline solution, and thus, a rhIDS solution of 0.1 mg/mL was prepared. A hemizygote mouse lacking a chromosomal region having an IDS gene (an IDS hemizygote mouse, C57BL/6N) was intravenously dosed with the rhIDS solution at a dosage of 0.5 mg/kg body weight, a total of four times every 7 days. The mouse was subjected to euthanasia with exsanguination after 7 days from the last dose. In this case, the blood was collected in a tube, and was left to stand at a room temperature for longer than or equal to 30 minutes, and then, was subjected to centrifugation (2000 g, for 20 minutes), and thus, the blood serum was recovered. Likewise, the blood serum was also recovered from a rhIDS-untreated IDS hemizygote mouse (a rhIDS-untreated IDS hemizygote mouse) and a wild type mouse.

In the blood serum collected from each of the mice, 2 μL of blood serum, 8 μL of PBS, and 18 μL of a citric acid buffer solution (pH 3.0) of 10 mM were added to two borosilicic acid screw-top test tubes, respectively, and were stirred. This was a blood sample solution. The blood sample solution was subjected to a methanolysis reaction in the condition B (a heating temperature of 80°C and a heating time of 2 hours) and the condition A (a heating temperature of 65°C and a heating time of 75 minutes) described in Example 9. Next, the blood sample solution after the methanolysis reaction was subjected to the LC/MS/MS analysis described in Example 10, and the dermatan sulfate and the heparan sulfate contained in the blood sample solution were quantified. Here, the dermatan sulfate was quantified by using a

sample subjected to the methanolysis reaction in the condition A, and the heparan sulfate was quantified by using a sample subjected to the methanolysis reaction in the condition B. The measurement was implemented by using 3 to 4 mice together with a wild type mouse, an IDS-treated hemizygote mouse, and an IDS-untreated hemizygote mouse.

Quantification results of the heparan sulfate and the dermatan sulfate in the blood sample solution are shown in Fig. 7(A) and Fig. 7(B), respectively. Both quantitative values of the heparan sulfate and the dermatan sulfate were higher in the rhIDS-untreated IDS hemizygote mouse than in the wild type mouse. In addition, in the rhIDS-treated IDS hemizygote mouse, both of the quantitative values of the heparan sulfate and the dermatan sulfate were reduced, compared to the rhIDS-untreated IDS hemizygote mouse. Such results indicate that the blood sample solution is treated by using the condition B (Heating Temperature: 80°C, Heating Time: 2 hours) as a methanolysis condition for quantifying the heparan sulfate, and by using the condition A (Heating Temperature: 65°C, Heating Time: 75 minutes) as a methanolysis condition for quantifying the dermatan sulfate, and thus, the heparan sulfate and dermatan sulfate contained in the blood can be quantified. Further, from the obtained quantitative values, it is indicated that the effect of an enzyme replacement therapy using IDS or a substance having IDS activity can be quantitatively verified.

[INDUSTRIAL APPLICABILITY]

According to the invention, the concentration of a small amount of chondroitin sulfate contained in a sample can be accurately measured, and thus, for example, the invention can be used in the screening of a patient with a lysosomal storage disease including a Sly syndrome and a Morquio syndrome in which chondroitin sulfate is accumulated in the body.

**Claims**

1. A method for decomposing chondroitin sulfate contained in a sample into disaccharide represented by formula (IV) below:

(Chem. 4)

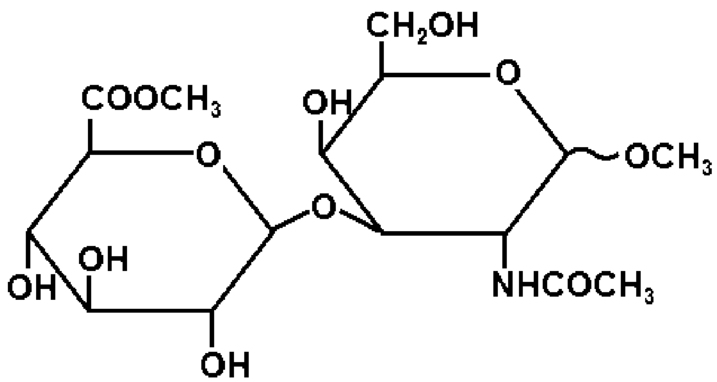

(IV)

wherein the chondroitin sulfate is decomposed by heating in HCl-methanol containing 2,2-dimethoxypropane at a temperature of 60°C to 90°C for 50 minutes to 180 minutes.

2. The method according to claim 1, wherein the heating for decomposing the chondroitin sulfate is performed at a temperature of 65°C to 75°C for 70 minutes to 110 minutes.

3. The method according to claim 1 or 2, wherein the sample is selected from a body fluid, a cell, a tissue, an organ, a cell culture medium, a tissue culture medium, a food, and a feed, or a derivative thereof.

4. The method according to claim 1 or 2, wherein the sample is selected from the group consisting of a body fluid, a cell, a tissue, an organ, blood, a blood serum, a blood plasma, urine, a bone marrow fluid, a cerebral spinal fluid, and a derivative thereof obtained from a mammal.

5. The method according to claim 4, wherein the mammal is selected from the group consisting of a human, a monkey, a mouse, a rat, a guinea pig, a hamster, a rabbit, a horse, a cow, a pig, a dog, and a cat.

**6.** The method according to claim 4, wherein the mammal is a human, and the human is a patient with a disorder in which chondroitin sulfate is accumulated in the body.

**7.** The method according to claim 6, wherein the disorder is selected from the group consisting of Maroteaux-Lamy syndrome, Morquio syndrome type A, Morquio syndrome type B, and Sly syndrome.

**8.** The method according to claim 6 or 7, wherein the patient has been treated to reduce the chondroitin sulfate present in the body.

**9.** A method for decomposing chondroitin sulfate and heparan sulfate contained in a sample, wherein the chondroitin sulfate is decomposed into disaccharide represented by formula (IV) below, and

(Chem. 4)

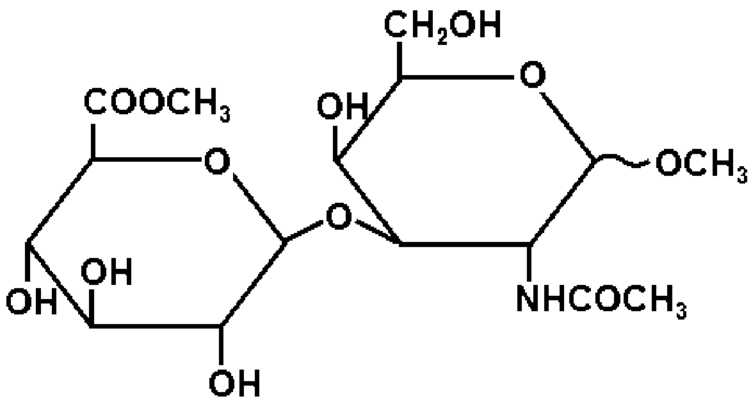

(IV)

the heparan sulfate is decomposed into disaccharide represented by formula (XIV) below, and

(Chem. 14)

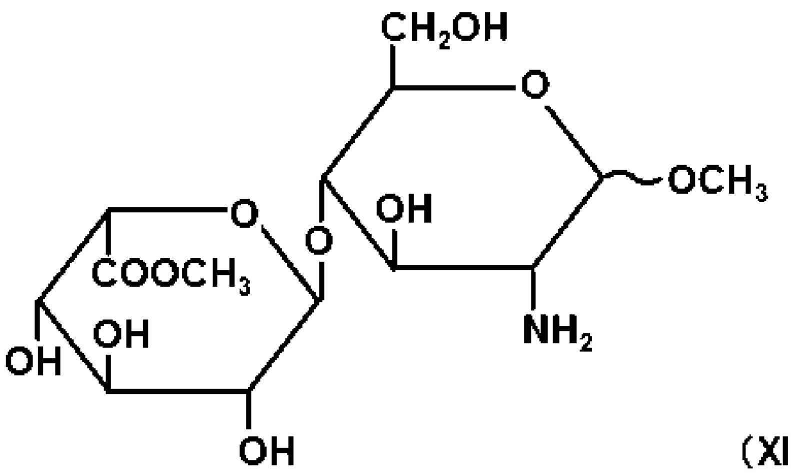

(XIV)

wherein the heparan sulfate is decomposed by heating in HCl-methanol containing 2,2-dimethoxypropane at a temperature of 65°C to 85°C for 80 minutes to 180 minutes, and the chondroitin sulfate is decomposed by the method according to claim 1 or 2.

**10.** The method according to claim 9, wherein the heating for decomposing the heparan sulfate is performed at a temperature of 78°C to 82°C for 110 minutes to 130 minutes.

**11.** A method for decomposing chondroitin sulfate, heparan sulfate, and dermatan sulfate contained in a sample, wherein the chondroitin sulfate is decomposed into disaccharide represented by formula (IV) below,

(Chem. 4)

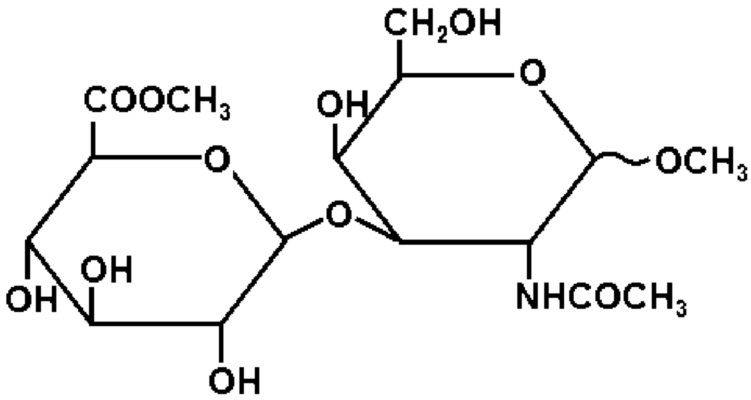

(IV)

the heparan sulfate is decomposed into disaccharide represented by formula (XIV) below, and

(Chem. 14)

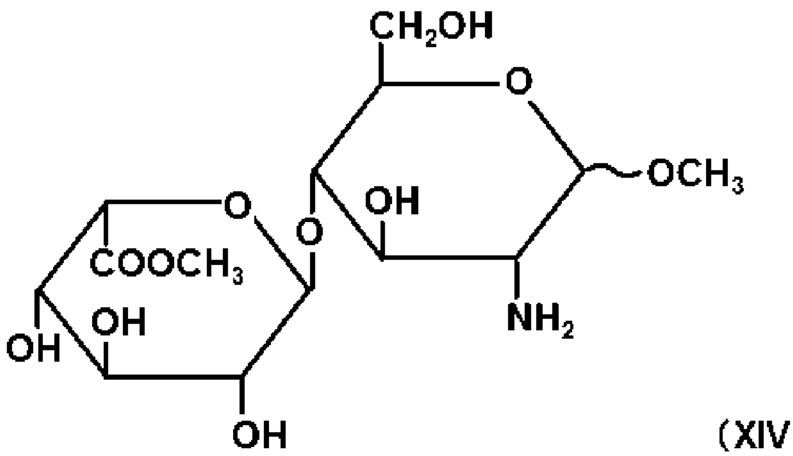

(XIV)

the dermatan sulfate is decomposed into disaccharide represented by formula (XII) below, and

(Chem. 12)

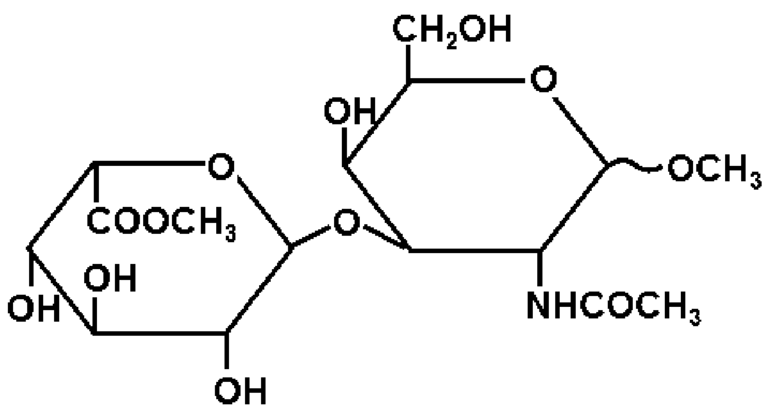

(XII)

wherein the dermatan sulfate is decomposed by heating in HCl-methanol containing 2,2-dimethoxypropane at a temperature of 60°C to 80°C for 20 minutes to 100 minutes, and the chondroitin sulfate and the heparan sulfate are decomposed by the method according to claim 9 or 10.

**12.** The method according to claim 11, wherein the heating for decomposing the dermatan sulfate is performed at a temperature of 63°C to 67°C for 30 minutes to 80 minutes.

**13.** The method according to any one of claims 9 to 12, wherein the sample is selected from a body fluid, a cell, a tissue, an organ, a cell culture medium, a tissue culture medium, a food, a feed, and the derivative thereof.

**14.** The method according to any one of claims 9 to 12, wherein the sample is selected from the group consisting of a body fluid, a cell, a tissue, an organ, a blood, a blood serum, a blood plasma, a urine, a bone marrow fluid, a cerebral spinal, and the derivative thereof obtained from a mammal.

**15.** The method according to claim 14, wherein the mammal is selected from the group consisting of a human, a monkey, a mouse, a rat, a guinea pig, a hamster, a rabbit, a horse, a cow, a pig, a dog, and a cat.

**16.** The method according to claim 14, wherein the mammal is a human, and the human is a patient with a disorder in which one or a plurality of chondroitin sulfate, heparan sulfate, and dermatan sulfate have been accumulated in the body.

**17.** The method according to claim 16, wherein the disorder is selected from the group consisting of Maroteaux-Lamy syndrome, Morquio syndrome type A, Morquio syndrome type B, Hunter syndrome, Hurler syndrome, Scheie syndrome, Hurler-Scheie syndrome, Sanfilippo syndrome, and Sly syndrome.

**18.** The method according to claim 16 or 17, wherein the patient has been treated to reduce one or a plurality of chondroitin sulfate, heparan sulfate, and dermatan sulfate present in the body.

**19.** A method for measuring the amount of chondroitin sulfate contained in a sample, the method including:

a step of obtaining an eluate by applying disaccharide obtained by the method according to any one of claims 1 to 8 to liquid chromatography; and
a step of applying the eluate to mass spectrometry.

**20.** A method for measuring the amount of chondroitin sulfate and heparan sulfate contained in a sample, the method including:

a step of obtaining an eluate by applying disaccharide obtained by decomposing chondroitin sulfate and heparan sulfate by the method according to claim 9 or 10 to liquid chromatography; and
a step of applying the eluate to mass spectrometry.

**21.** A method for measuring the amount of chondroitin sulfate, heparan sulfate, and dermatan sulfate contained in a sample, the method including:

a step of obtaining an eluate by applying disaccharide obtained by decomposing chondroitin sulfate, heparan sulfate, and dermatan sulfate by the method according to any one of claims 10 to 18 to liquid chromatography; and
a step of applying the eluate to mass spectrometry.

**22.** A method for detecting an individual with a disorder in which chondroitin sulfate is accumulated in the body from among a mammal providing a sample, the sample obtained by the method according to claim 19, on the basis of a measurement value of chondroitin sulfate contained in the sample.

**23.** A method for detecting an individual with a disorder in which chondroitin sulfate and/or heparan sulfate is accumulated in the body from among a mammal providing a sample, the sample obtained by the method according to claim 20, on the basis of a measurement value of chondroitin sulfate and/or heparan sulfate contained in the sample.

**24.** A method for detecting an individual with a disorder in which one or a plurality of chondroitin sulfate, heparan sulfate, and dermatan sulfate are accumulated in the body from a mammal providing a sample, the sample obtained by the method according to claim 21, on the basis of measurement values of chondroitin sulfate, heparan sulfate, and dermatan sulfate.

**25.** The method according to any one of claims 22 to 24, wherein the disorder is selected from the group consisting of

Maroteaux-Lamy syndrome, Morquio syndrome type A, Morquio syndrome type B, Hunter syndrome, Hurler syndrome, Scheie syndrome, Hurler-Scheie syndrome, Sanfilippo syndrome, and Sly syndrome.

**26.** A method for confirming an effect of a medical treatment, on the basis of a measurement value obtained by measuring the amount of a substance accumulated in a body and contained in samples by the method according to any one of claims 19 to 21,
wherein the samples are obtained from a patient, the patient suffering from a disorder in which one or a plurality of chondroitin sulfate are accumulated in the body and receiving a medical treatment to reduce the amount of the substance in the body, before and after receiving the treatment.

[FIG. 1/7]

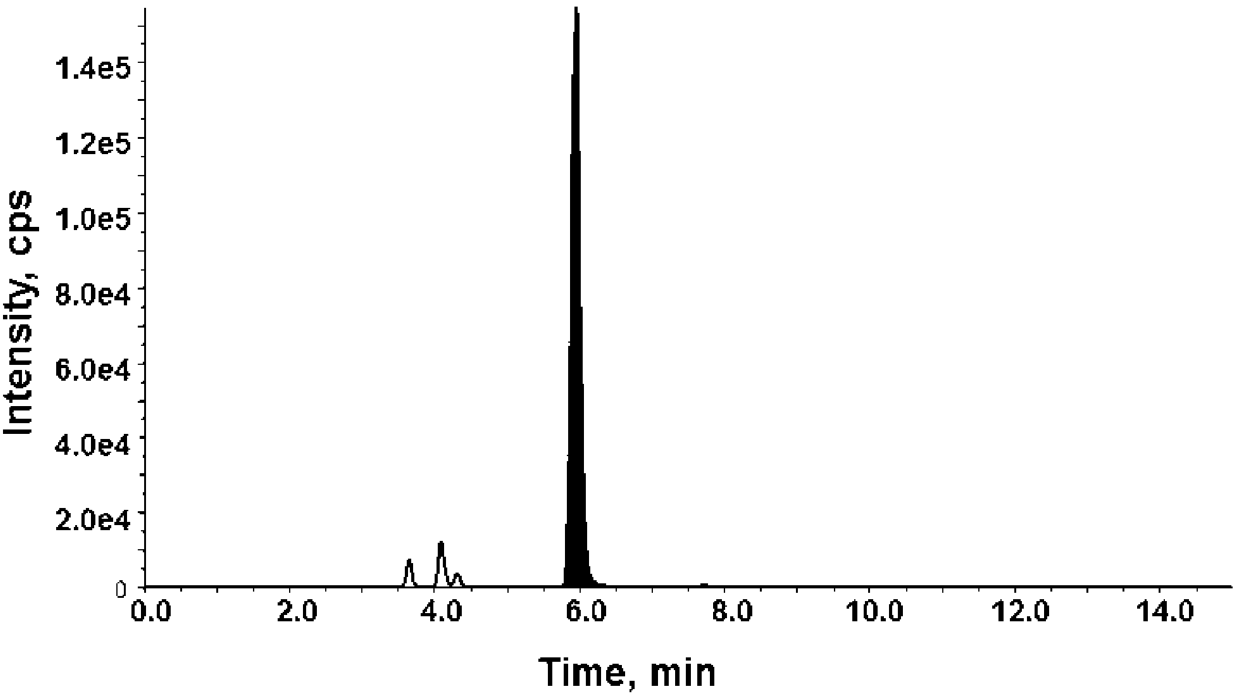


[FIG. 2/7]

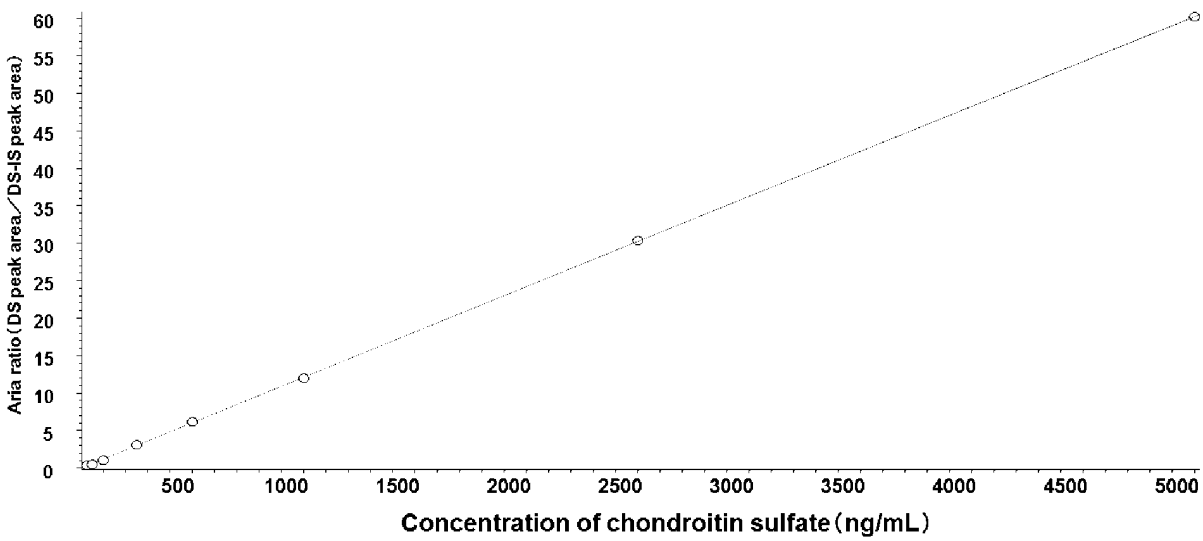


[FIG. 3/7]

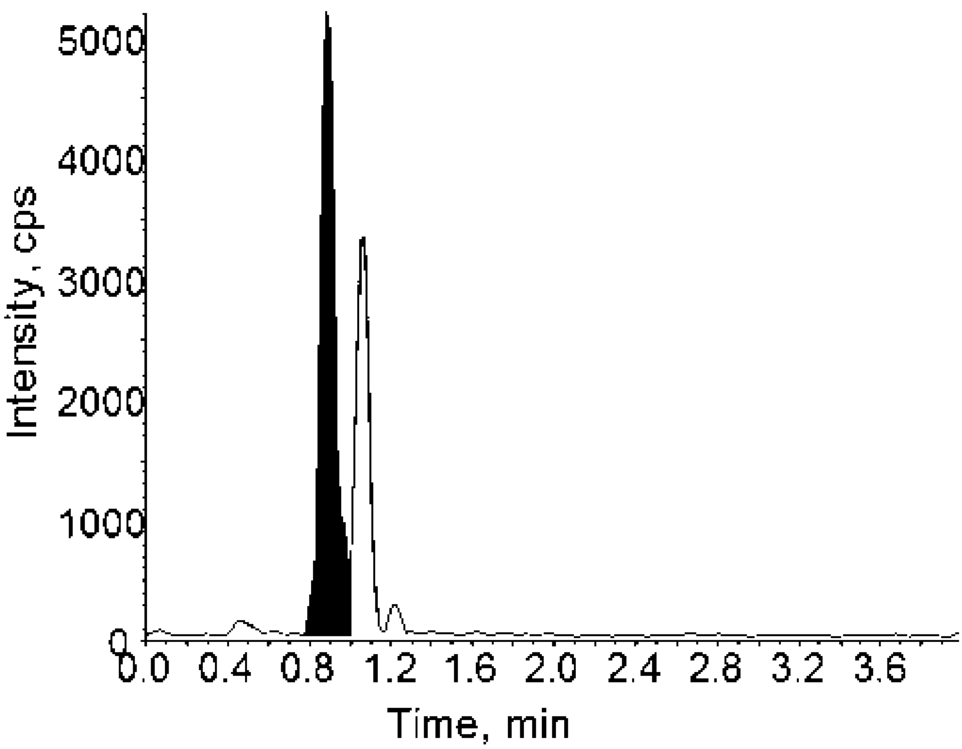
5000
4000
3000
2000
1000
0
Intensity, cps
0.0 0.4 0.8 1.2 1.6 2.0 2.4 2.8 3.2 3.6
Time, min

[FIG. 4/7]

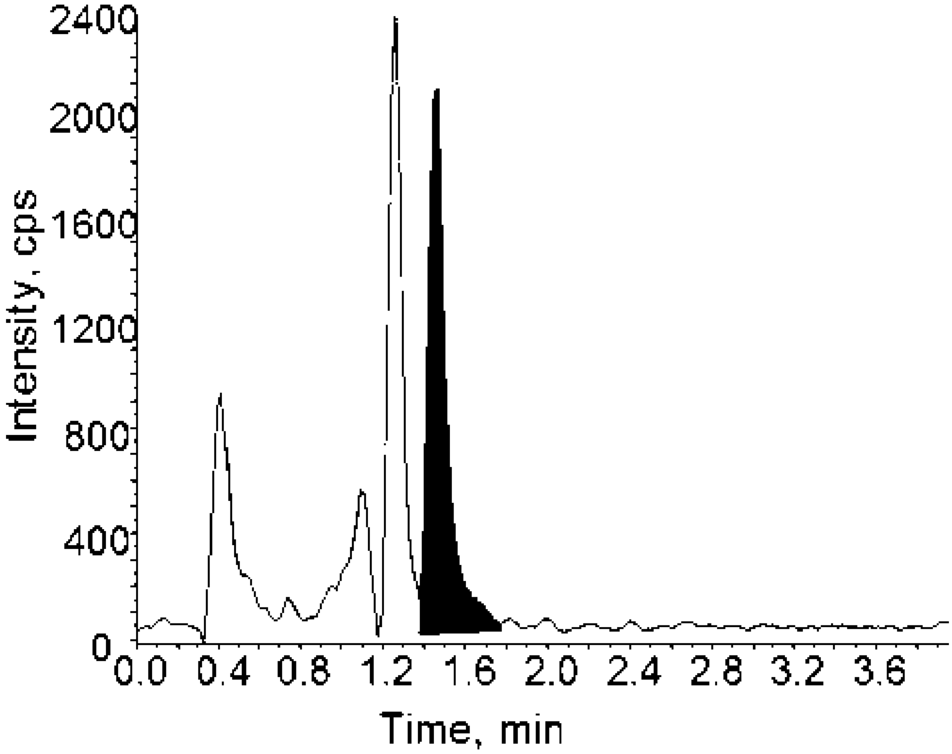
2400
2000
1600
1200
800
400
0
Intensity, cps
0.0 0.4 0.8 1.2 1.6 2.0 2.4 2.8 3.2 3.6
Time, min

[FIG. 5/7]

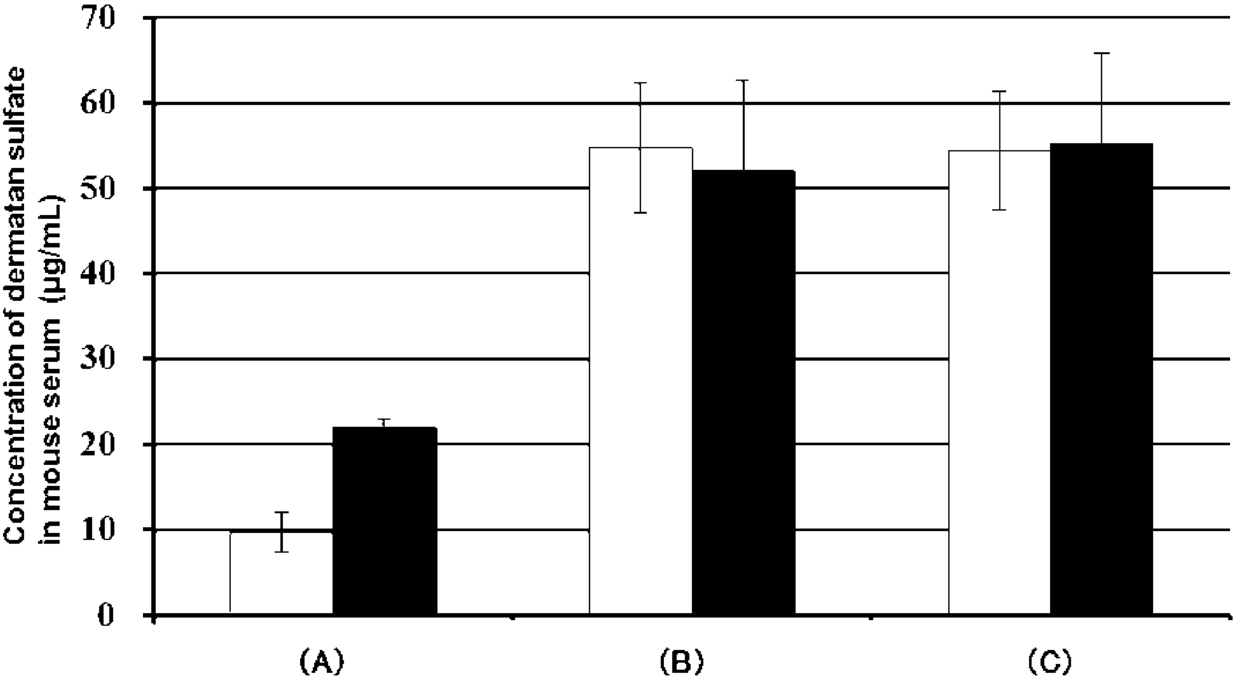
70
60
50
40
30
20
10
0
Concentration of dermatan sulfate in mouse serum (µg/mL)
(A)
(B)
(C)

[FIG. 6/7]

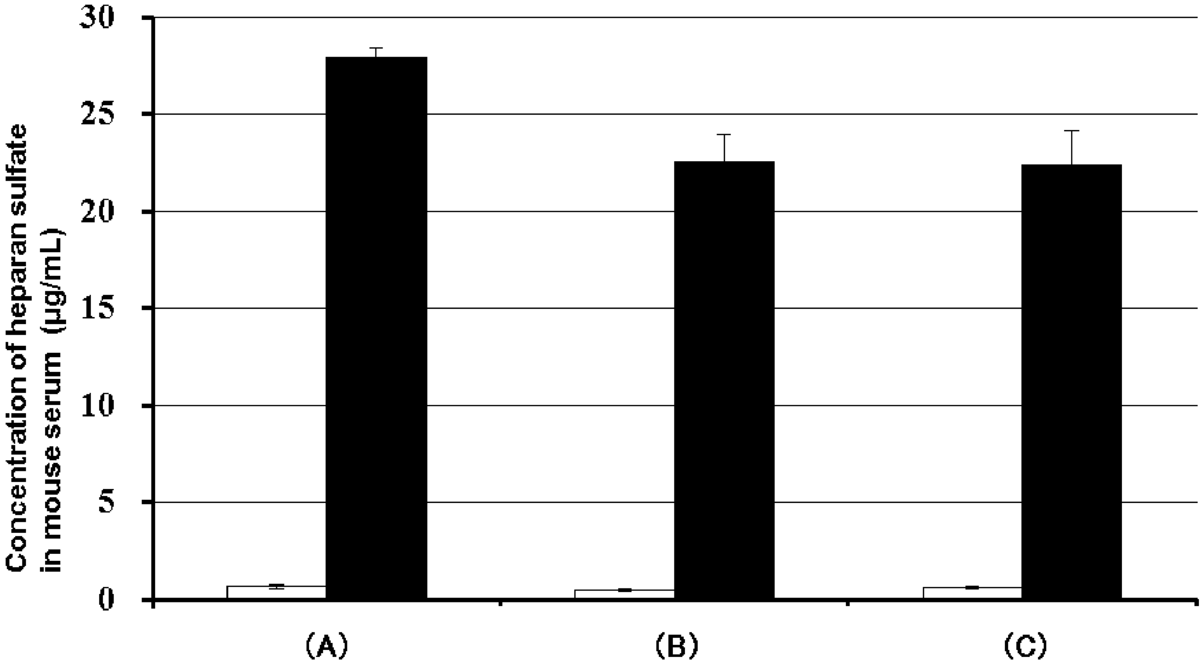
30
25
20
15
10
5
0
Concentration of heparan sulfate in mouse serum (µg/mL)
(A)
(B)
(C)

[FIG. 7/7]

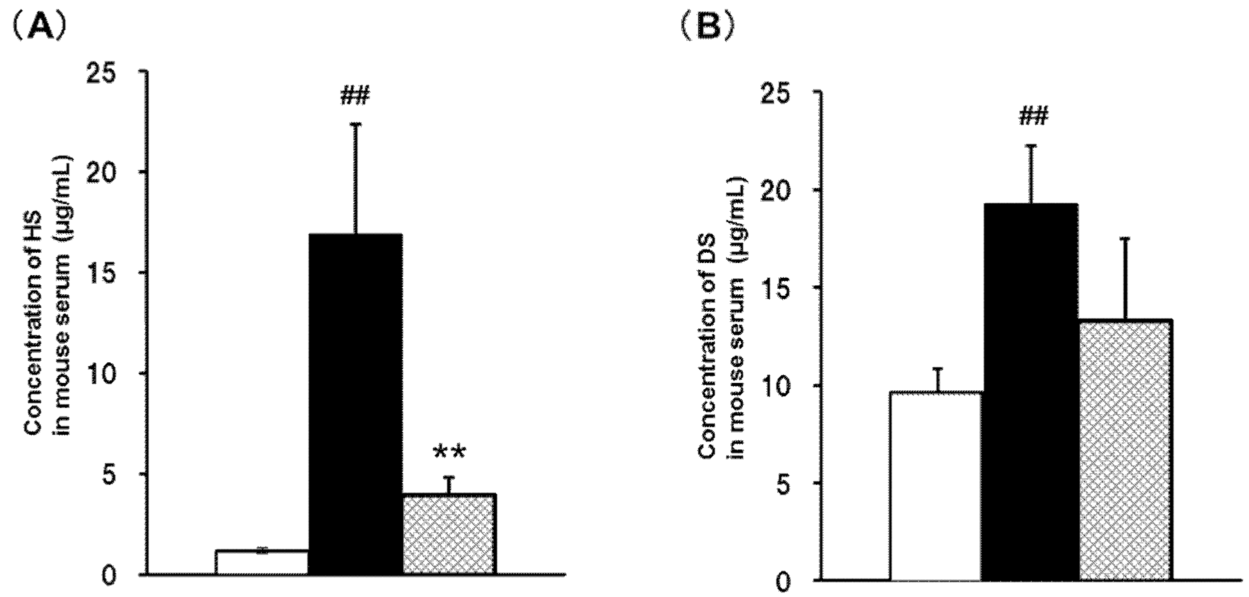
(A)
25
20
15
10
5
0
Concentration of HS in mouse serum (μg/mL)
##
**
(B)
25
20
15
10
5
0
Concentration of DS in mouse serum (μg/mL)
##

**INTERNATIONAL SEARCH REPORT**

International application No. PCT/JP2019/032218

A. CLASSIFICATION OF SUBJECT MATTER
Int.Cl. G01N33/48(2006.01)i, C07H5/06(2006.01)i, G01N27/62(2006.01)i, G01N30/72(2006.01)i, G01N33/66(2006.01)i

According to International Patent Classification (IPC) or to both national classification and IPC

B. FIELDS SEARCHED

Minimum documentation searched (classification system followed by classification symbols)
Int.Cl. G01N33/48, C07H5/06, G01N27/62, G01N30/72, G01N33/66

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| A | JP 2012-108056 A (CELLSEED INC.) 07 June 2012 & US 2013/0309703 A1 & WO 2012/067236 A1 & EP 2642286 A1 | 1-26 |
| A | JP 2017-535774 A (SHIRE HUMAN GENETIC THERAPIES, INC.) 30 November 2017 & US 2017/0350900 A1 & WO 2016/077775 A1 & EP 3218722 A1 & AU 2015346064 A1 & CN 107003324 A & CA 2967382 A1 | 1-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 November 2019 (01.11.2019) | 19 November 2019 (19.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/032218

C (Continuation). DOCUMENTS CONSIDERED TO BE RELEVANT

| Category* | Citation of document, with indication, where appropriate, of the relevant passages | Relevant to claim No. |
|---|---|---|
| A | AURAY-BLAIS, Christiane et al., "UPLC-MS/MS detection of disaccharides derived from glycosaminoglycans as biomarkers of mucopolysaccharidoses", Analytica Chemica Acta, 14 September 2016, vol. 936, PP. 139-148 | 1-26 |
| A | AURAY-BLAIS, C. et al., "Efficient analysis of urinary glycosaminoglycans by LC-MS/MS in mucopolysaccharidoses type I, II and VI", Molecular Genetics and Metabolism, January 2011, vol. 102, Issue 1, 49-56 | 1-26 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012108056 A


### Non-patent literature cited in the description


- **AURAY-BLAIS C. et al.** *Mol Genet Metab.,* 2011, vol. 102, 49-56
- **AURAY-BLAIS C. et al.** *Clin Chim Acta.,* 2012, vol. 413, 771-8
- **ZANG H. et al.** *Clin Chem.,* 2011, vol. 57, 1005-12